(19) Europäisches Patentamt / European Patent Office / Office européen des brevets

(11) **EP 4 714 337 A1**

(12) **EUROPEAN PATENT APPLICATION**

(43) Date of publication:
25.03.2026 Bulletin 2026/13

(21) Application number: 25203266.9

(22) Date of filing: 19.09.2025

(51) International Patent Classification (IPC):
A61B 5/00 (2006.01)        A61B 5/024 (2006.01)
A61B 5/103 (2006.01)       G06T 7/00 (2017.01)
G06V 10/56 (2022.01)       G06V 40/10 (2022.01)

(52) Cooperative Patent Classification (CPC):
A61B 5/0077; A61B 5/0022; A61B 5/0035;
A61B 5/02444; A61B 5/1032; A61B 5/6893;
A61B 5/7485; G06T 7/0012; G06V 10/56;
G06V 40/15; A61B 2576/00; G06T 2207/10024

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 19.09.2024 KR 20240126409

(71) Applicant: GB Soft Co., Ltd.
Daegu 42117 (KR)

(72) Inventor: PARK, Ki Bum
42117 Daegu (KR)

(74) Representative: BCKIP Part mbB
MK1
Landsbergerstraße 98, 3.Stock
80339 München (DE)

(54) **ELECTRONIC DEVICE FOR MEASURING RPPG BASED ON PLURALITY OF COLOR MODELS AND PROVIDING SERVICE, AND OPERATION METHOD OF THE SAME**

(57) According to various embodiments, there is provided an operation method of an electronic device including: acquiring a plurality of images of a user, acquiring a specific area on a specific body part of the user from the plurality of images, generating a plurality of first data associated with an RGB color model on the specific area associated with the plurality of images, generating a plurality of second data associated with a YCrCb color model based on the plurality of first data, generating first time-series data associated with a green channel based on the plurality of first data, generating second time-series data associated with a color difference channel based on the plurality of second data, generating integrated time-series data based on combining the first time-series data and the second time-series data, and estimating a pulse rate of the user based on conversion of the integrated time-series data into a frequency domain.

*FIG.5*

START

ACQUIRE PLURALITY OF IMAGES OF USER, CAPTURED BY RGB CAMERA(501)

IDENTIFY SPECIFIC AREAS FROM PLURALITY OF IMAGES (503)

GENERATE PLURALITY OF FIRST DATA ASSOCIATED WITH FIRST COLOR MODEL ON SPECIFIC AREAS OF PLURALITY OF IMAGES (505)

GENERATE PLURALITY OF SECOND DATA ASSOCIATED WITH SECOND COLOR MODEL BASED ON PLURALITY OF FIRST DATA (507)

GENERATE FIRST TIME-SERIES DATA ASSOCIATED WITH GREEN CHANNEL BASED ON PLURALITY OF FIRST DATA (509)

GENERATE SECOND TIME-SERIES DATA ASSOCIATED WITH COLOR DIFFERENCE CHANNEL BASED ON PLURALITY OF SECOND DATA (511)

GENERATE AT LEAST ONE INTEGRATED TIME-SERIES DATA ASSOCIATED WITH COMBINING FIRST TIME-SERIES DATA AND SECOND TIME-SERIES DATA (513)

ACQUIRE BIOMETRIC INFORMATION OF USER BASED ON INTEGRATED TIME-SERIES DATA (515)

END

EP 4 714 337 A1

## Description

### CROSS-REFERENCE TO RELATED APPLICATION

This application claims priority to and the benefit of Korean Patent Application No. 10-2024-0126409, filed on September 19, 2024, the disclosure of which is incorporated herein by reference in its entirety.

### BACKGROUND

#### 1. Field of the Invention

The disclosure relates to an electronic device for measuring remote photoplethysmography (rPPG) based on a plurality of color models and providing services, and an operation method thereof.

#### 2. Discussion of Related Art

The most common technology for measuring photoplethysmography (PPG) by using light uses a method of analyzing an amount of light transmitted in response to light projected onto a human body, and is explained by the Beer-Lambert law stating that absorbance is proportional a concentration of an absorbing material and a thickness of an absorbing layer. According to this law, a change in transmitted light results in a signal that is proportional to a change in the volume of a material through which light is transmitted, and hence, when the absorbance of a material is not known, a state of heart, etc. may be identified by using PPG.

Recently, going a step further from the technology using PPG, technologies using rPPG are emerging. As a most popularized technology for identifying signals related to heart beat using PPG, there is a method of obtaining PPG by bringing a device having a camera and a light attached nearby, such as a smartphone, into contact with a human body directly and irradiating light, and immediately measuring transmitted light. Recently, technologies related to rPPG for identifying a change in the volume of a blood vessel from signals obtained from a captured image by a camera are being researched and developed.

Technologies using rPPG are variously applied in devices and places provided with cameras, such as airport immigration offices, telemedicine hospitals, etc. since they do not require contact between a target object and a measurement device.

However, since the rPPG technology has the great influence of ambient light and noises caused by motions of a target object on signals in the process of photographing the target object with a camera, the technique of extracting only signals related to a change in the volume of a measurement target object from a photographed image may be regarded as a core technology in measuring biometric signals using rPPG.

## SUMMARY OF THE INVENTION

According to various embodiments, there may be provided an operation method of an electronic device including: acquiring a plurality of images of a user, captured by an RGB camera of the electronic device; acquiring a specific area on a specific body part of the user from the plurality of images; generating a plurality of first data associated with an RGB color model on the specific area associated with the plurality of images; generating a plurality of second data associated with a YCrCb color model based on the plurality of first data; generating first time-series data associated with a green channel based on the plurality of first data; generating second time-series data associated with a color difference channel based on the plurality of second data; generating third time-series data based on combining the first time-series data and the second time-series data; and estimating a pulse rate of the user based on conversion of the third time-series data into a frequency domain.

According to various embodiments, there may be provided an electronic device including at least one processor, wherein the at least one processor is configured to: acquire a plurality of images of a user, captured by an RGB camera; acquire a specific area on a specific body part of the user from the plurality of images; generate a plurality of first data associated with an RGB color model on the specific area associated with the plurality of images; generate a plurality of second data associated with a YCrCb color model based on the plurality of first data; generate first time-series data associated with a green channel based on the plurality of first data; generate second time-series data associated with a color difference channel based on the plurality of second data; generate third time-series data based on combining the first time-series data and the second time-series data; and estimate a pulse rate of the user based on conversion of the third time-series data into a frequency domain.

The technical solution according to various embodiments is not limited to that mentioned above, and other technical solutions that are not mentioned above may be clearly understood to those skilled in the art based on the detailed descriptions and the accompanying drawings.

When rPPG is measured in a related-art method, it is common to analyze an image of a captured target object by using a camera, and in this case, rPPG may be measured based on pixel values of the captured image which are extracted based on various color models, such as a RGB color model, a YCbCrCg color model, a CIELa*b* color model, etc. However, since noise values caused by a photographing environment (for example, shaking, external illuminance, shadow, etc.) are reflected on the pixel values when photographing is performed by the camera, there may be difficulty in accurately measuring rPPG of the target object. According to various embodiments, an electronic device and an operation method thereof may enhance accuracy of measurement of rPPG on a target object by measuring rPPG by using a plurality of color models including an RGB color model, a YCbCrCg color model, a CIELa*b* color model, etc. in the complementary form to reduce noise values. According to various embodiments, the electronic device and the operation method thereof may enhance accuracy of measurement of rPPG on a target object based on a color model value and an infrared ray (IR) value by using an RGB camera and an IR camera simultaneously.

Recently, technologies are developing for vehicles or manpower management kiosks placed at doorways to provide various services based on biometric information measured by using rPPG. However, due to low accuracy of rPPG and poor knowledge in methods of associating with services provided by vehicles or manpower management kiosks, substantially underutilized services may be provided. According to various embodiments, the electronic device and the operation method thereof may provide substantially highly-utilized vehicles and manpower management kiosk services based on high-quality rPPG which is measurable in all environments.

According to various embodiments, there may be provided an electronic device and an operation method thereof which enhance accuracy of measurement of rPPG on a target object by measuring rPPG by using a plurality of color models including an RGB color model, a YCbCrCg color model, a CIELa*b* color model, etc. in the complementary form to reduce noise values.

According to various embodiments, there may be provided an electronic device and an operation method thereof which enhance accuracy of measurement of rPPG on a target object based on a color model value and an IR value by using an RGB camera and an IR camera simultaneously.

According to various embodiments, there may be provided an electronic device and an operation method thereof which provide substantially highly-utilized vehicles and manpower management kiosk services based on high-quality rPPG which is measurable in all environments.

**BRIEF DESCRIPTION OF THE DRAWINGS**

FIG. 1 is a view illustrating examples of components of a non-contact biometric information system according to various embodiments;
FIG. 2 is a view illustrating other examples of components of a non-contact biometric information system according to various embodiments;
FIG. 3 is a block diagram illustrating example of components of a server according to various embodiments;
FIG. 4 is a block diagram illustrating examples of components of a user device according to various embodiments;

FIG. 5 is a flowchart provided to explain an example of an operation method of an electronic device which measures rPPG and biometric information based on a plurality of color models according to various embodiments;
FIG. 6 is a view provided to explain examples of modules which perform operations for measuring rPPG and biometric information by using a visible ray camera according to various embodiments;
FIG. 7 is a view provided to explain an example of an operation of pre-processing RGB data according to various embodiments;
FIG. 8 is a view provided to explain an example of an operation of using a plurality of color models according to various embodiments;
FIG. 9 is a flowchart provided to explain an example of an operation method of an electronic device for measuring rPPG and biometric information based on IR channel values for each of a plurality of areas according to various embodiments;
FIG. 10 is a view provided to explain examples of modules which perform operations for measuring rPPG and biometric information by using an infrared ray (IR) camera according to various embodiments;
FIG. 11 is a view provided to explain an example of an operation of using values of IR channels for each of a plurality of areas according to various embodiments;
FIG. 12 is a flowchart provided to explain an example of an operation method of an electronic device for measuring rPPG by using a visible ray camera and an IR camera based on a photographing environment condition according to various embodiments;
FIGS. 13A to 13C are a view provided to explain embodiments for measuring rPPG by using a visible ray camera and an IR camera according to various embodiments;
FIG. 14 is a flowchart provided to explain an example of an operation method of an electronic device for measuring a blood pressure based on a blood pressure AI model according to various embodiments;
FIG. 15 is a flowchart provided to explain an example of an operation method of an electronic device for measuring a stress based on a stress analysis AI model according to various embodiments;
FIG. 16 is a view provided to explain examples of modules for performing a stress analysis operation according to various embodiments;
FIG. 17 is a view provided to explain an example of a transportation means for providing a service based on non-contact biometric information according to various embodiments;
FIG. 18 is a flowchart provided to explain an example of an operation method of an electronic device for providing a mobility service based on non-contact biometric information and outside information according to various embodiments;
FIGS. 19A and 19B are a view provided to explain an example of a mobility service which is provided according to non-contact biometric information and outside information according to various embodiments;

FIG. 20 is a flowchart provided to explain an example of an operation method of an electronic device for providing a mobility service further considering passenger information according to various embodiments;

FIG. 21 is a view provided to explain an example of measurement of rPPG/biometric information on a passenger according to various embodiments;

FIG. 22 is a flowchart provided to explain an example of an operation method of an electronic device for providing a mobility service further considering a mode of a vehicle according to various embodiments;

FIG. 23 is a view provided to explain an example of a mobility service which continuously deforms a configuration of a seat during autonomous driving according to various embodiments;

FIG. 24 is a flowchart provided to explain an example of an operation method of an electronic device for providing a continuous biometric information accumulation-based service according to various embodiments;

FIG. 25A is a view provided to explain an embodiment for accumulating biometric information measured for each driving path for a pre-set time in a database according to various embodiments;

FIG. 25B is a view provided to explain an example of a service which provides driving recommendation information, based on the accumulated biometric information according to various embodiments;

FIG. 26 is a view provided to explain an example of a kiosk which provides a service based on non-contact biometric information according to various embodiments;

FIG. 27 is a flowchart provided to explain an example of an operation method of an electronic device for providing a service based on biometric information at each of a plurality of locations according to various embodiments;

FIG. 28 is a view provided to explain an example of collecting biometric information on a specific user from a kiosk which is placed at each of a plurality of locations according to various embodiments; and

FIGS. 29A and 29B are a view provided to explain an example of a service based on biometric information of each of a plurality of locations according to various embodiments.

## DETAILED DESCRIPTION OF EXEMPLARY EMBODIMENTS

It should be appreciated that various embodiments of the disclosure and the terms used therein are not intended to limit the technological features set forth herein to particular embodiments and include various changes, equivalents, or alternatives for a corresponding embodiment. With regard to the description of the drawings, similar reference numerals may be used to refer to similar or related elements. It is to be understood that a singular form of a noun corresponding to an item may include one or more of the items, unless the relevant context clearly indicates otherwise. As used herein, each of such phrases as "A or B," "at least one of A and B," "at least one of A or B," "A, B, or C," "at least one of A, B, and C," and "at least one of A, B, or C," may include at least one or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiment of the disclosure may be implemented as software (e.g., a program) including one or more instructions that are stored in a storage medium (e.g., internal memory) that is readable by a machine (e.g., the electronic device). For example, a processor (e.g., a processor) of the machine (e.g., an electronic device) may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in other components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to various embodiments, there may be provided an operation method of an electronic device including: acquiring a plurality of images of a user, captured by an RGB camera of the electronic device; acquiring a specific area on a specific body part of the user from the plurality of images; generating a plurality of first data associated with an RGB color model on the specific area associated with the plurality of images; generating a plurality of second data associated with a YCrCb color model based on the plurality of first data; generating first time-series data associated with a green channel based on the plurality of first data; generating second time-series data associated with a color difference channel based on the plurality of second data; generating third time-series data based on combining the first time-series data and the second time-series data; and estimating a pulse rate of the user based on conversion of the third time-series data into a frequency domain.

According to various embodiments, the acquiring the specific area on the specific body part of the user from the plurality of images may include: identifying values on a specific color model on the plurality of images; and extracting an area corresponding to a range of a pre-set value among the identified values.

According to various embodiments, the generating the plurality of first data associated with the RGB color model may include: acquiring a plurality of values for each of a plurality of color channels of the RGB color model for each of the plurality of images, each of the plurality of values corresponding to a specific time point; identifying a plurality of interval values of a plurality of time windows among the plurality of values of the plurality of color channels; and generating the plurality of first data by adjusting the plurality of values based on an average of the plurality of interval values.

According to various embodiments, there may be provided the operation method further including performing at least one of a noise removal operation by using a signal filter or a trend correction operation with respect to the plurality of first data.

According to various embodiments, the generating the first time-series data associated with the green channel may include: generating first sub time-series data by subtracting a value of a red channel from a value of the green channel, and generating second sub time-series data by subtracting a value of a blue channel from the value of the green channel; and generating the first time-series data by combining the first sub time-series data and the second sub time-series data.

According to various embodiments, there may be provided the operation method further including: generating the plurality of second data for the YCrCb color model from the plurality of first data; and generating the second time-series data by combining third sub time-series data of a Cr channel and fourth sub time-series data of a Cb channel based on the plurality of second data.

According to various embodiments, there may be provided an electronic device including at least one processor, wherein the at least one processor may be configured to: acquire a plurality of images of a user, captured by an RGB camera; acquire a specific area on a specific body part of the user from the plurality of images; generate a plurality of first data associated with an RGB color model on the specific area associated with the plurality of images; generate a plurality of second data associated with a YCrCb color model based on the plurality of first data; generate first time-series data associated with a green channel based on the plurality of first data; generate second time-series data associated with a color difference channel based on the plurality of second data; generate third time-series data based on combining the first time-series data and the second time-series data; and estimate a pulse rate of the user based on conversion of the third time-series data into a frequency domain.

According to various embodiments, the at least one processor may be configured to, as at least a part of the operation of acquiring the specific area on the specific body part of the user from the plurality of images: identify values on a specific color model on the plurality of images; and extract an area corresponding to a range of a pre-set value among the identified values.

According to various embodiments, the at least one processor may be configured to, as at least a part of the operation of generating the plurality of first data associated with the RGB color model: acquire a plurality of values for each of a plurality of color channels of the RGB color model for each of the plurality of images, each of the plurality of values corresponding to a specific time point; identify a plurality of interval values of a plurality of time windows among the plurality of values of the plurality of color channels; and generate the plurality of first data by adjusting the plurality of values based on an average of the plurality of interval values.

According to various embodiments, the at least one processor may further be configured to perform at least one of a noise removal operation by using a moving average filter, a high frequency noise removal operation, or a trend correction operation using detrend with respect to the plurality of first data.

## 1. Non-contact biometric information system (1)

A non-contact biometric information system 1 according to various embodiments may be a system which is implemented to provide various services by measuring rPPG of a target object based on an image on the target object photographed by a camera, and obtaining biometric information (for example, blood pressure, pulse rate, stress index, etc.) measured based on the rPPG. The rPPG may refer to an amount of blood (PPG) measured from blood vessels in the proximity of skin in a non-contact method (that is, in a remote method). The non-contact biometric information system 1 may enhance quality of measured rPPG by reducing the influence of noises by a photographing environment (for example, illuminance, shadow, shaking) reflected on a captured image by the camera, and accordingly, may provide services of high practicality. Hereinafter, the disclosure will be described in detail.

## 2. Components of the non-contact biometric information system (1)

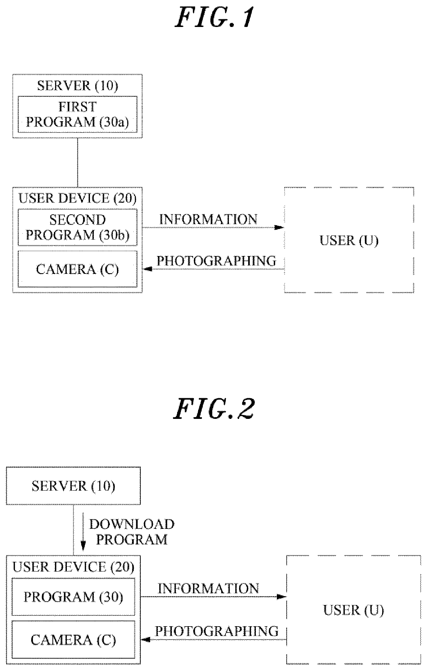

FIG. 1 is a view illustrating examples of components of the non-contact biometric information system 1 according to various embodiments.

Referring to FIG. 1, the non-contact biometric information system 1 according to various embodiments may include a server 10 and a user device 20. All of the server 10 and the user device 20 may be defined as an "electronic device".

According to various embodiments, the server 10 may be implemented to measure rPPG and biometric information attributable thereto based on an image on a user U (for example, an image on a part of body) photographed by a camera C of the user device 20, and to provide various types of services. For example, referring to FIG. 1, the server 10 may store a first program 30a, may measure rPPG of the user U and biometric information attributable thereto, based on the image on the user received from the user device 20, based on the first program 30a, and may provide various types of services. The first program 30a may be implemented to include at least one of a software module, a program, a variety of information (parameter), or an artificial intelligence (AI) model for analyzing the image of the user U.

According to various embodiments, the user device 20 may be an electronic device of the user U. The user device 20 may include not only an electronic device carried by the user U, such as a smartphone, a tablet, a laptop, a wearable device, a head-mounted display (HMD) device, or the like, but also an installable electronic device such as a kiosk, a personal computer (PC), a television (TV), or the like, but is not limited thereto and may further include various types of electronic devices including a camera to include hardware for photographing the user U. The user device 20 may display a graphic user interface which is implemented to provide a photographing function on the user U to the user U based on execution of a second program 30b (for example, may display an execution screen providing a photographing function), and may provide at least one of rPPG, biometric information, or information on various types of services received from the server 10.

FIG. 2 is a view illustrating other examples of components of a non-contact biometric information system 1 according to various embodiments. Referring to FIG. 2, the non-contact biometric information system 1 may include a server 10 and a user device as shown in FIG. 1, but only a program 30 may be implemented. The program 30 may include all of the first program 30a and the second program 30b described above, and accordingly, the function of the server 10 described above may be operated independently (or in the form of on-device) in the user device 20. In other words, the user device 20 may be implemented to capture an image on the user U by using a camera C, to measure rPPG and biometric information attributable thereto by analyzing the captured image, and to provide various types of services, based on execution of the program 30.

It is obvious to those skilled in the art that operations of the electronic device according to various embodiments, which will be described below, are understood as operations of the server 10, operations of the user device 20, or operations by cooperation of the server 10 and the user device 20.

## 2.1 Components of the electronic device

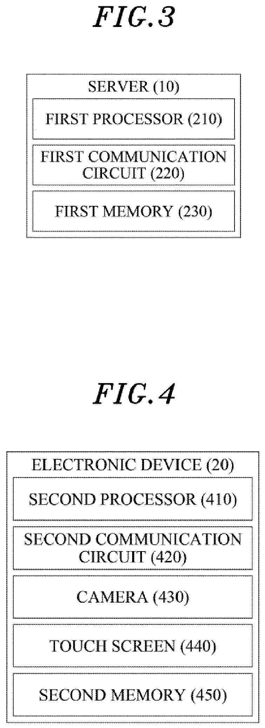

Hereinafter, examples of components of electronic devices constituting the non-contact biometric information system 1 according to various embodiments will be described with reference to FIGS. 3 and 4.

## 2.1.1 Components of the server 10

FIG. 3 is a block diagram illustrating examples of components of the server 10 according to various embodiments.

Referring to FIG. 3, the server 10 according to various embodiments may include a first processor 210, a first communication circuit 220, and a first memory 230. However, the disclosure is not limited to examples described and/or illustrated, and the server 10 may include more components.

According to various embodiments, the first processor 210 may control overall operations of the server 10. To achieve this, the first processor 210 may compute and process a variety of information, and may control operations of the components (for example, the first communication circuit 220) of the server 10. According to an embodiment, as at least a part of data processing or computation, the first processor 210 may store a command or data received from another component in a volatile memory, and may process a command or data stored in the volatile memory, and may store resulting data in a nonvolatile memory. According to an embodiment, the first processor 210 may include a main processor (not shown) (for example, a central processing unit or an application processor) or an auxiliary processor (not shown) (for example, a graphic processing unit, a neural processing unit (NPU), an image signal processor, a sensor hub processor, or a communication processor) operating independently therefrom or cooperatively therewith. For example, when the server 10 includes the main processor (not shown) and the auxiliary processor (not shown), the auxiliary processor (not shown) may be set to use lower power than the main processor (not shown), or to be specific to a designated function. The auxiliary processor (not shown) may be implemented separately from the main processor (not shown), or as a part thereof.

According to an embodiment of the present application, the auxiliary processor (not shown) may control at least a part of functions or states related to at least one component (for example, the first communication circuit 220) among the components of the server 10, on behalf of the main processor (not shown) during an inactive (for example, sleep) state of the main processor (not shown), or along with the main processor (not shown) during an active state (for example, an application execution state) of the main processor (not shown). According to an embodiment, the auxiliary processor (not shown) (for example, the image signal processor or the communication processor) may be implemented as a part of other components (for example, the first communication circuit 220) functionally related thereto. According to an embodiment, the auxiliary processor (not shown) (for example, the neural processing unit) may include a hardware structure which is specific to processing by an AI model. The AI model may be generated through machine learning. Such learning may be performed in the server 10 in which AI is performed, or may be performed through a separate server (for example, a learning server). For example, a learning algorithm may include, but not be limited to, supervised learning, unsupervised learning, semi-supervised learning or reinforcement learning. The AI model may include a plurality of artificial neural network layers. The artificial neural network may include, but not be limited to, a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or one of combinations of the two or more networks. The AI model may include a software structure in addition to or alternatively to a hardware structure.

In the following descriptions, operations of the server 10 may be interpreted as being performed under control of the first processor 20 unless mentioned otherwise.

According to various embodiments, the first communication circuit 220 may communicate with an external device (for example, the user device 20). For example, the first communication circuit 220 may be connected to a network through wireless communication or wired communication to set communication with the external device (for example, the user device 20), and may exchange information and/or data through the set communication. The wireless communication may include, for example, cellular communication using at least one of LTE, LTE-Advance (LTE-A), code division multiple access (CDMA), wideband CDMA (WCDMA), universal mobile telecommunications system (UMTS), wireless broadband (WiBro), or global system for mobile communications (GSM). According to an embodiment, the wireless communication may include at least one of wireless fidelity (WiFi), Bluetooth, Bluetooth low energy (BLE), Zigbee, near field communication (NFC), magnetic secure transmission, radio frequency (RF), or body area network (BAN). According to an embodiment, the wireless communication may include a Global Navigation Satellite System (GNSS). The GNSS may be, for example, a global positioning system (GPS), a GLObal NAvigation Satellite System (GLONASS), a Beidou navigation satellite system (hereinafter, "Beidou") or Galileo, the European global satellite-based navigation system. In the disclosure, "GPS" may be interchangeably used with "GNSS". The wired communication may include, for example, at least one of a universal serial bus (USB), a high definition multimedia interface (HDMI), recommended standard 232 (RS-232), low power line communication or a plain old telephone service (POTS), etc. The network may include a telecommunication network, for example, at least one of a computer network (for example, LAN or WAN), Internet, or a telephone network.

According to an embodiment of the present application, the memory 230 may store a variety of information. The memory 230 may temporarily or semi-permanently store data. For example, the memory 230 may store an authoring module 200 to create an active experience file. The server 10 (for example, the first processor 210) may perform an operation for creating an active experience file, based on the authoring module 200

200.

## 2.1.2 Components of the user device 20

FIG. 4 is a block diagram illustrating examples of components of the user device 20 according to various embodiments.

According to various embodiments, referring to FIG. 4, the user device 20 may include a second processor 410, a second communication circuit 420, a camera 430, a touch screen 440, and a second memory 550. The second processor 410 may be implemented like the above-described first processor 210, the second communication circuit 420 may be implemented like the above-described first communication circuit 220, and the second memory 450 may be implemented like the first memory 230, and thus redundant explanations are omitted.

According to various embodiments, the camera 430 may be implemented to capture an image on a user of the user device 20. For example, the camera 430 may include at least one of a visible ray camera (or an RGB camera) or an infrared ray (IR) camera. The visible ray camera may be a camera which is capable of capturing an image of a normal visible ray area (for example, a wavelength band of 380 to 780 nm) (that is, capturing an image based on visible rays), and the IR camera may be a camera which is capable of capturing an image of an IR area (for example, a wavelength area of 700 nm to 1000 nm) (that is, capturing an image based on infrared rays). According to the type of the user device 20, the user device 20 may be implemented in a form including the visible ray camera, the IR camera or all of the visible ray camera and the IR camera. When the user device is implemented in the form including all of the visible ray camera and the IR camera, the user device 20 may measure rPPG based on an image of the visible ray area and an image of the IR area, which will be described in detail below.

According to various embodiments, the touch screen 440 may be implemented to display a graphic user interface including predetermined information, and to acquire user's input received on the graphic user interface. For example, the user device 20 (for example, the second processor 410) may display a graphic user interface (for example, an execution screen) including at least one of rPPG, biometric information or various types of services received from the server 10. In another example, the user device 20 (for example, the second processor 410) may display a graphic user interface (for example, an execution screen) including a menu screen and/or icon for controlling the camera 430, and may activate the camera 430 based on a user's input on the icon being received through the touch screen 440 to capture an image on the user.

## 3. rPPG and biometric information measurement method

## 3.1. rPPG and biometric information measurement based on a plurality of color models

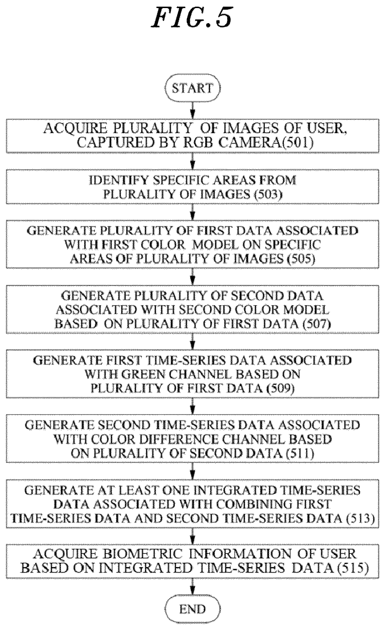

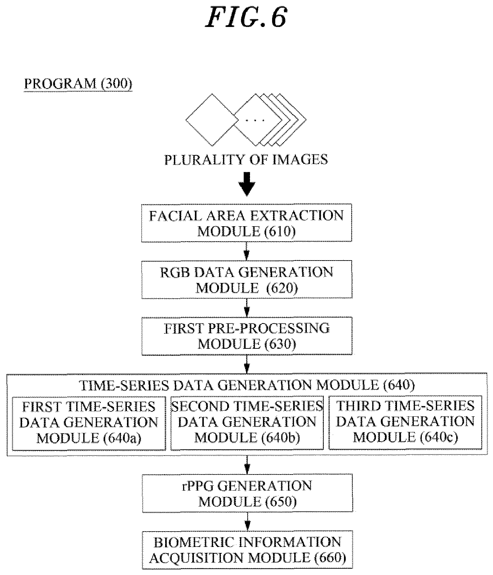

FIG. 5 is a flowchart provided to explain an example of an operation method of an electronic device (for example, the server 10, the user device 20) for measuring rPPG and biometric information based on a plurality of color models according to various embodiments. However, operations may be performed in order different from the order of operations described and/or illustrated, and more operations or fewer operations than the operations described and/or illustrated may be performed. Hereinbelow, FIG. 5 will be described with reference to FIGS. 6 to 8.

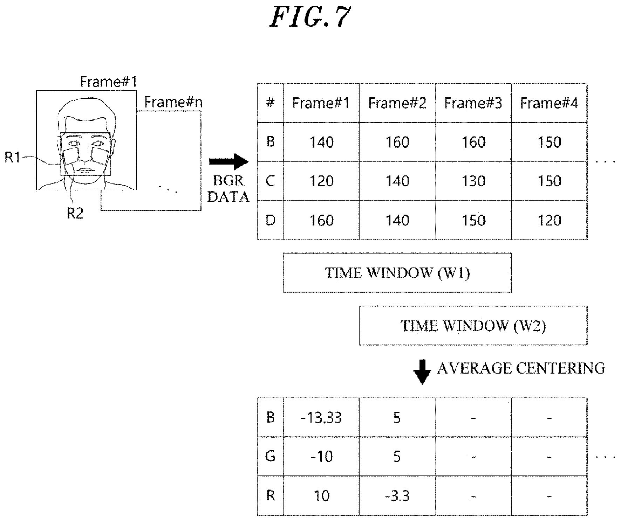

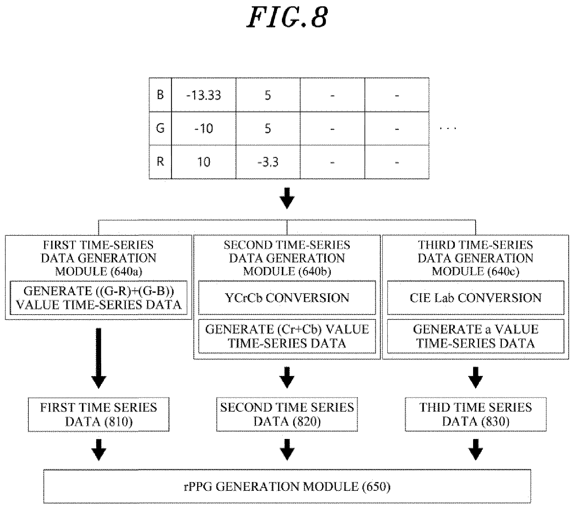

FIG. 6 is a view provided to explain examples of modules for performing operations for measuring rPPG and biometric information by using a visible ray camera according to various embodiments. FIG. 7 is a view provided to explain an example of an operation for pre-processing RGB data according to various embodiments. FIG. 8 is a view provided to explain an example of an operation of using a plurality of color models according to various embodiments.

According to various embodiments, the electronic device (for example, the server 10, the user device 20) may acquire a plurality of images of a user, captured by an RGB camera in operation 501, may identify specific areas from the plurality of images in operation 503, and may generate a plurality of first data associated with a first color model on the specific areas of the plurality of images in operation 505. For example, the electronic device (for example, the server 10, the user device 20) may acquire RGB data on a specific body part (for example, face) of the user for a pre-set time by using a visible ray camera among the cameras 430. For example, the user device 20 may capture a plurality of images of the user for the pre-set time by using the visible ray camera according to a user request. Referring to FIGS. 6 to 7, the electronic device (for example, the server 10, the user device 20) may identify a skin area R2 within a facial area R1 of each of the plurality of images (or image frames) Frame #1, ... , Frame #n captured by the user device 20 by using a facial area extraction module 610, and may acquire a value on an RGB color model on the skin area R2 by using an RGB data generation module 620, based on execution of a program 300 (for example, the second program 30b or the program 30). The disclosure is not limited to the above-described example, and a skin area of another body part (for example, thigh, forearm) other than the skin area R2 within the user's facial area R1 may be extracted.

According to various embodiments, as at least a part of the operation of identifying the skin area within the face by using the facial area extraction module 610, the electronic device (for example, the server 10, the user device 20) may identify the facial area R1 from the plurality of captured images Frame #1, ... , Frame #n based on an object identification algorithm, may convert RGB color model values for each of a plurality of pixels included in the identified facial area R1 into YCrCb color model values or HSV color model values, and then, may identify at least some pixels greater than or equal to a preset value as the skin area R2 within the facial area R1.

According to various embodiments, as at least a part of the operation of acquiring the values on the RGB color model on the skin area R2 by using the RGB data generation module 620, the electronic device (for example, the server 10, the user device 20) may remove noises by applying a Gaussian blur to the skin areas R2 of the plurality of images Frame #1, ⋯⋯ , Frame #n, and may acquire an average value of a B channel (blue channel) of the plurality of pixels included in the skin areas R2 of the plurality of images Frame #1, Frame #2, Frame #3, Frame #4, ⋯ , Frame #n) from which noises are removed, an average value of a R channel (red channel), and an average value of a G channel (green channel) (that is, an average value for each channel of the RGB color model on the skin areas R2). Referring to FIG. 7, the electronic device (for example, the server 10, the user device 20) may perform average centering with respect to the average value for each channel of the RGB color model on the skin areas R2 of the plurality of images (Frame #1, ⋯ , Frame #n). For example, the electronic device (for example, the server 10, the user device 20) may adjust (or change or control or correct) (that is, average centering) the average value for each channel of the RGB color model on the skin areas R2 of some images of the plurality of images Frame #1, ⋯ , Frame #n included in time windows W1, W2, based on an average value of each of BGR channels during as many time windows W1, W2 as the pre-set number of images (or pre-set time). For example, average centering may be performed on the RGB color model on the skin areas R2 according to Equation 1 presented below:

$$[Equation\ 1]\quad V'a = Va - \frac{\left(\frac{\sum_{i=1}^{n} Vi}{n}\right)}{m}$$

where $V'a$ is a value of a specific channel (B channel, G channel, R channel) of a mean-centered specific image frame, $Va$ is a value of a specific channel of a specific image frame before average centering, $Vi$ is a value of a specific channel of each image frame included in a specific time window, $i$ is an identification number of an image frame within a specific time window, $n$ is the number of image frames included in a specific time window, and $m$ is the number of time windows overlapping a specific image frame.

That is, according to the Equation 1, the average value of the specific channel of the specific time window included in the specific image may be subtracted from the value of the specific channel on the skin area R2 of the specific image. In this case, referring to FIG. 7, a plurality of time windows may be implemented and may be set to overlap one another, and accordingly, when the plurality of time windows are set for the specific image, an average value of the specific channel of the plurality of time windows may be subtracted from the value of the specific channel on the skin area R2 of the specific image, the average value of the specific channel subtracted may be divided by the number (m) of the plurality of time windows overlapping one another and may be subtracted.

The electronic device (for example, the server 10, the user device 20) may perform at least one of an amplitude correction or a trend correction based on the average value other than average centering.

According to various embodiments, referring to FIG. 6, the electronic device (for example, the server 10, the user device 20) may perform a pre-processing operation with respect to the RGB color model value on the skin area R2 of the plurality of images, by using a first pre-processing module 630. The pre-processing operation may include at least one of an operation of removing noises (for example, a high frequency noise, a low frequency noise) by using a signal filter (for example, a moving average filter), or a trend correction operation including detrending.

According to various embodiments, in operation 507, the electronic device (for example, the server 10, the user device 20) may generate a plurality of second data associated with a second color model (for example, YCrCb, CIE La*b*) based on the plurality of first data (for example, the RGB color model values on the skin area R2). For example, referring to FIGS. 6 and 8, the electronic device (for example, the server 10, the user device 20) may convert RGB color model values on the skin area R2 on the plurality of images into values on other color models by using a time-series data generation module 640 (for example, first to third time-series data generation modules 640a, 640b, 640c). The other color models may include at least one of the YCrCb color model or the CIE La*b* color model, but is not limited to the above-described example.

According to various embodiments, the electronic device (for example, the server 10, the user device 20) may generate first time-series data associated with the green channel, based on the plurality of first data (for example, the RGB color model values) in operation 509, may generate at least one second time-series data associated with a color difference channel based on the plurality of second data (for example, the YCrCb color model values) in operation 511, and may generate integrated time-series data based on combining the first time-series data and the second time-series data in operation 513. For example, the electronic device (for example, the server 10, the user device 20) may generate final time-series data (for example, integrated time-series data) by combining values of the plurality of color models (for example, the RGB color model, the YCrCb color model or the CIE La*b* color model) on the skin area R2 acquired as a result. The disadvantages of different color models are compensated for by the combination, so that noises caused by a photographing environment (for example, illuminance, shadow, shaking) when a user is photographed can be reduced and rPPG of enhanced quality can be acquired.

In an embodiment, referring to FIG. 8, the electronic device (for example, the server 10, the user device 20) may generate first time-series data 810 by using the first time-series data generation module 640a by adding a first value which is the red channel subtracted from the green channel and a second value which is the blue channel subtracted from the green channel for each of the plurality of images by using the RGB color model values on the skin area R2. The green channel best reflects the amount of blood, and there may be an error by the red channel and the blue channel included in an ambient light source during photographing. Accordingly, the first time-series data value which is the red channel and the blue channel subtracted from the green channel may reflect the amount of blood well, and may reduce an error by the red channel and the blue channel of the external light source.

In an embodiment, referring to FIG. 8, the electronic device (for example, the server 10, the user device 20) may generate second time-series data 810 by using the second time-series data generation module 640b by adding all of the color difference channels (Cr channel, Cb channel) for each of the plurality of images by using the YCrCb color model values on the skin area R2

In an embodiment, referring to FIG. 8, the electronic device (for example, the server 10, the user device 20) may generate third time-series data 830 including only an *a* value by using the third time-series data generation module 640c by using the CIE La*b* color model values on the skin area R2.

The electronic device (for example, the server 10, the user device 20) may generate third time-series data by using a rPPG generation module 650 by combining at least a part of the plurality of time-series data 810, 820, 830 generated. For example, the electronic device (for example, the server 10, the user device 20) may generate integrated time-series data by combining the first time-series data 810 and the second time-series data 820. Accordingly, the value of the first time-series data 810 from which the red channel and the blue channel are removed may be complemented by the second time-series data 820 including the color difference channel. The electronic device (for example, the server 10, the user device 20) may generate integrated time-series data by adding the first time-series data 810, the second time-series data 820, and the third time-series data 830. The electronic device (for example, the server 10, the user device 20) may generate integrated time-series data by combining the second time-series data 820 and the third time-series data 830. The generated integrated time-series data indicates a blood flow rate and may be defined as a rPPG signal.

According to various embodiments, the electronic device (for example, the server 10, the user device 20) may perform a pre-processing operation with respect to the plurality of time-series data 810, 820, 830 before combining the plurality of time-series data 810, 820, S830. For example, the pre-processing operation may include a Z-score operation.

According to various embodiments, in operation 515, the electronic device (for example, the server 10, the user device 20) may acquire biometric information of the user based on the integrated time-series data. For example, the electronic device (for example, the server 10, the user device 20) may acquire the biometric information by analyzing the integrated time-series data by using a biometric information acquisition module 660. The biometric information may include a pulse rate, a blood pressure, a stress index, etc. For example, in operation 515, the electronic device (for example, the server 10, the user device 20) may convert the integrated time-series data into a frequency domain, may extract a frequency value of a frequency domain corresponding to a pulse rate range (40 bpm to 240 bpm), may extract a pre-set frequency band (for example, a frequency band corresponding to 20 bpm) including the highest frequency among the extracted frequency values by using a band pass filter, and may extract a pulse rate based on an inter-peak distance in the extracted frequency band. The electronic device (for example, the server 10, the user device 20) may estimate other biometric information such as a blood pressure, etc., based on the extracted pulse rate.

### 3.2 rPPG and biometric information measurement based on IR channel values for each of a plurality of areas

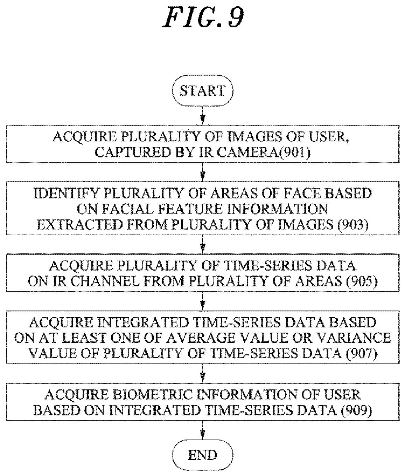

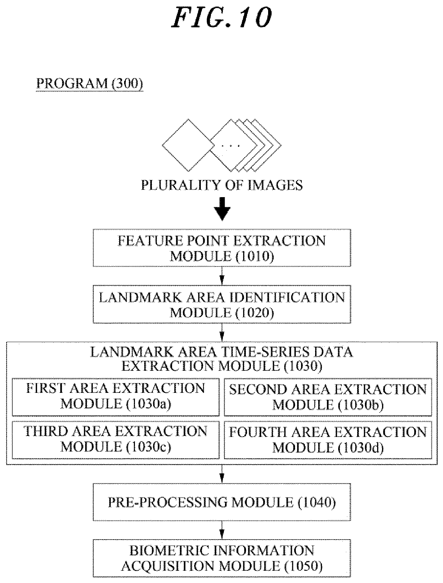

FIG. 9 is a flowchart provided to explain an example of an operation method of an electronic device (for example, the server 10, the user device 20) for measuring rPPG and biometric information based on IR channel values for each of a plurality of areas according to various embodiments. However, operations may be performed in order different from the order of operations described and/or illustrated, and more operations or fewer operations than the operations described and/or illustrated may be performed. Hereinbelow, FIG. 9 will be described in detail with reference to FIGS. 10 to 11.

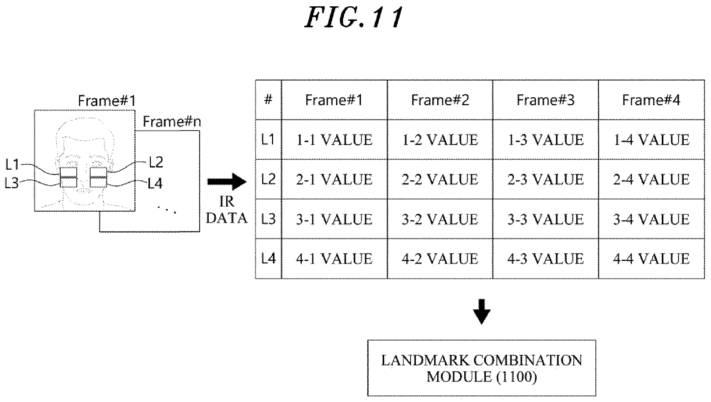

FIG. 10 is a view provided to explain examples of modules for performing an operation of measuring rPPG and biometric information by using an IR camera according to various embodiments. FIG. 11 is a view provided to explain an example of an operation of using values of IR channels for each of a plurality of areas according to various embodiments.

According to various embodiments, in operation 901, the electronic device (for example, the server 10, the user device 20) may acquire a plurality of images of a user, captured by an IR camera, and for example, the electronic device (for example, the server 10, the user device 20) may acquire IR data on a specific body part (for example, face) of the user for a pre-set time by using the IR camera among the cameras 430 to measure rPPG on the user. For example, the user device 20 may capture a plurality of images of the user for a pre-set time by using the IR camera according to a user request.

According to various embodiments, in operation 903, the electronic device (for example, the server 10, the user device 20) may identify a plurality of areas of the face based on facial feature information extracted from the plurality of images. For example, referring to FIGS. 10 and 11, the electronic device (for example, the server 10, the user device 20) may extract feature points indicating the user's face from the plurality of images by using a feature point extraction module 1010, and may identify, as a plurality of landmark areas L1, L2, L3, L4, the areas on feature points defined as landmarks among the extracted feature points, by using a landmark identification module 1020. For example, the plurality of landmark areas L1, L2, L3, L4 may include a left cheek upper portion L1, a left cheek lower portion L3, a right cheek upper portion L2 and a right cheek lower portion L4. The landmark identification module 1020 may be an AI model that is trained to identify feature points indicating portions (for example, specific skeleton) defining the plurality of landmark areas L1, L2, L3, L4, respectively, and may identify and output feature points indicating the portions (for example, specific skeleton) defining the landmark areas L1, L2, L3 L4, respectively, based on information (for example, coordinates, vector) on the extracted feature points being inputted. The electronic device (for example, the server 10, the user device 20) may define areas on the outputted feature points as the landmark areas L1, L2, L3, L4.

According to various embodiments, the electronic device (for example, the server 10, the user device 20) may acquire a plurality of time-series data on IR channels from the plurality of areas L1, L2, L3, L4 in operation 905, and may acquire integrated time-series data based on at least one of an average value or a variance value of the plurality of time-series data in operation 907. For example, referring to FIG. 10, the electronic device (for example, the server 10, the user device 20) may extract average time-series signals of IR channels of the plurality of areas L1, L2, L3, L4 by using a landmark area time-series data extraction module 1030 (for example, first to fourth area extraction modules 1030a, 1030b, 1030c, 1030d), may pre-process the average time-series signals of the IR channels of the plurality of areas L1, L2, L3, L4 extracted, and may acquire integrated time-series data by combining the IR channel signals of the plurality of areas L1, L2, L3, L4 pre-processed.

For example, as at least a part of the operation of pre-processing the average time-series signals of the IR channels, the electronic device (for example, the server 10, the user device 20) may use a plurality of area extraction modules 1030a, 1030b, 1030c, 1030d. For example, the electronic device (for example, the server 10, the user device 20) may generate average time-series signals of IR channels of the plurality of areas L1, L2, L3, L4 on the plurality of image frames captured in sequence by using a deque, by using the plurality of area extraction modules 1030a, 1030b, 1030c, 1030d, and may generate time-series signals on IR channels of the plurality of areas L1, L2, L3, L4 by performing at least one of average centering or Z-score on the average time-series signals of the IR channels of the plurality of areas L1, L2, L3, L4 generated. The generated time-series signals may be defined as rPPG signals. Thereafter, the electronic device (for example, the server 10, the user device 20) may pre-process by using a pre-processing module 1040 by performing at least one of moving average filter/moving time window-based detrending or Bezier curve-based filtering with respect to rPPG signals of the plurality of areas L1, L2, L3, L4.

As a result, the electronic device (for example, the server 10, the user device 20) may generate integrated time-series data by combining some of the rPPG signals on the IR channels of the plurality of areas L1, L2, L3, L4 pre-processed. For example, the combination may refer to generating the integrated time-series data by performing the four fundamental arithmetic operations on the rPPG signals on the IR channels of the plurality of areas L1, L2, L3, L4 pre-processed, and the combination may be determined according to a photographing environment (for example, illuminance, shadow, shaking). For example, the integrated time-series data may be generated by adding a first result signal which is the rPPG of the second area L2 subtracted from the rPPG of the first area L1 under a first photographing environment condition (for example, a shadow condition), and a second result signal which is the rPPG of the fourth area L4 subtracted from the rPPG of the third area L3, and the integrated time-series data may be generated by adding a third result signal which is the rPPG of the third area L3 subtracted from the rPPG of the first area L1 under a second photographing environment condition (for example, a shadow condition), and a fourth result signal which is the rPPG of the fourth area L4 subtracted from the rPPG of the second area L2. The disclosure is not limited to the example described above, and rPPG signals on the IR channels of the plurality of areas L1, L2, L3, L4 pre-processed may be combined in various methods.

According to various embodiments, in operation 909, the electronic device (for example, the server 10, the user device 20) may acquire biometric information of the user based on the integrated time-series data. For example, the electronic device (for example, the server 10, the user device 20) may estimate a pulse rate by interpreting a frequency on the integrated time-series data as in operation 515 described above, by using a biometric information acquisition module 1050, and a redundant explanation is omitted.

For example, the electronic device (for example, the server 10, the user device 20) may acquire RGB data on a specific body part of the user (for example, face) for a pre-set time by using a visible ray camera among the cameras 430 to measure rPPG on the user. For example, the user device 20 may capture a plurality of images of the user for a pre-set time by using the visible ray camera according to a user request. Referring to FIGS. 6 and 7, the electronic device (for example, the server 10, the user device 20) may identify a skin area R2 within a facial area R1 of each of the plurality of images (or image frames) Frame #1, ··· , Frame #n captured by the user device 20 by using the facial area extraction module 610, and may acquire values on the RGB color model on the skin area R2 by using the RGB data generation module 620, based on execution of the program 300 (for example, the second program 30b or the program 30). The disclosure is not limited to the above-described example, and a skin area may be extracted from other body parts (for example, thigh, forearm) other than the skin area R2 in the facial area R1 of the user.

### 3.3. Hybrid rPPG and biometric information measurement

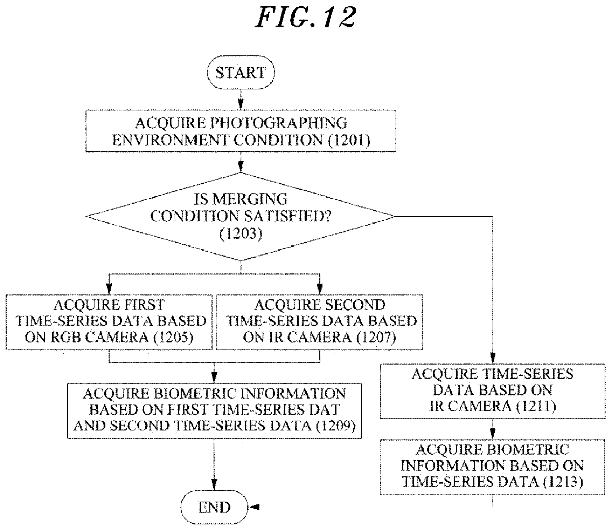

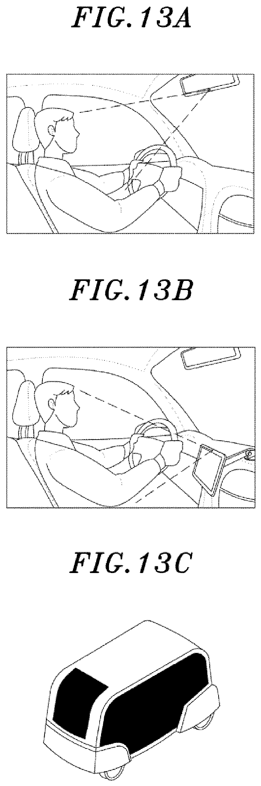

FIG. 12 is a flowchart provided to explain an example of an operation method of an electronic device (for example, the server 10, the user device 20) for measuring rPPG by using a visible ray camera and an IR camera based on a photographing environment condition according to various embodiments. However, operations may be performed in order different from the order of operations described and/or illustrated, and more operations or fewer operations than the operations described and/or illustrated may be performed. Hereinbelow, FIG. 12 will be described in detail with reference to FIG 13.

FIGS. 13A to 13C are a view provided to explain embodiments for measuring rPPG by using a visible ray camera and an IR camera according to various embodiments.

According to various embodiments, the electronic device (for example, the server 10, the user device 20) may acquire a photographing environment condition in operation 1201, and may determine whether a merging condition is satisfied in operation 1203. For example, non-contact rPPG/biometric information measurement and service may be performed under various photographing conditions. Accordingly, the electronic device (for example, the server 10, the user device 20) may acquire information that influences photographing by a camera, such as information on current illuminance, information on shaking, as a photographing environment condition, and may determine whether the merging condition is satisfied, based on identifying whether the acquired information exceeds (or is less than) a pre-set value. For example, when an illuminance value exceeds a pre-set value, it may be determined that the merging condition is satisfied, and, when the illuminance value is less than the pre-set value, it may be determined that the merging condition is not satisfied. For example, when a shaking value exceeds a pre-set value, it may be determined that the merging condition is satisfied, and, when the shaking value is less than the pre-set value, it may be determined that the merging condition is not satisfied.

For example, referring to FIGS. 13A to 13C, non-contact rPPG/biometric information measurement and service may be performed on a passenger (for example, a driver or a passenger) in a transportation means. Referring to FIG. 13A, non-contact rPPG/biometric information measurement and service may be performed based on a captured image by a camera provided in a vehicle, and, referring to FIG. 13B, non-contact rPPG/biometric information measurement and service may be performed based on a captured image by a camera of the user device 20 (for example, a mobile device) held in a vehicle. Accordingly, the electronic device (for example, the server 10, the user device 20) may acquire, as information on the photographing environment condition, information that influences photographing by the camera, such as information on illuminance, information on shaking, etc., which is acquired by a sensor (for example, an illuminance sensor, a motion sensor) provided in a transportation means or a sensor (for example, an illuminance sensor, a motion sensor) of the user device 20, and may determine whether the merging condition is satisfied based on the information. Referring to FIG. 13C, the transportation means may be an autonomous driving transportation means which does not require a driver (for example, an autonomous driving vehicle, an autonomous driving ship).

Although not shown in the example, cameras for non-contact rPPG/biometric information measurement and service may be provided in various places (or various spaces) provided with cameras, such as house, a building, an office, a corridor, etc. in addition to the transportation means.

According to various embodiments, when the merging condition is satisfied (1203-Y), the electronic device (for example, the server 10, the user device 20) may acquire first time-series data based on an RGB camera in operation 1205, may acquire second time-series data based on an IR camera in operation 1207, and may acquire biometric information based on the first time-series data and the second time-series data in operation 1209. For example, when the merging condition is satisfied, the photographing environment may be recognized as an environment in which the accuracy of rPPG is degraded, and accordingly, the electronic device (for example, the server 10, the user device 20) may generate first integrated time-series data based on images captured by the visible ray camera according to the operation method of FIG. 5 described above, and may generate second integrated time-series data based on images captured by the IR camera according to the operation method of FIG. 9. The electronic device (for example, the server 10, the user device 20) may finally generate time-series data by reflecting the first and second integrated time-series data on each other (for example, summing and averaging, or simply summing), and may measure biometric information (for example, a pulse rate) based on the generated time-series data or may measure final biometric information by complementing the biometric information (for example, a pulse rate) measured based on the first and second integrated time-series data each other.

According to various embodiments, when the merging condition is not satisfied (1203-N), the electronic device (for example, the server 10, the user device 20) may acquire time-series data based on the RGB camera in operation 1211, and may acquire biometric information based on the time-series data in operation 1213. For example, the electronic device (for example, the server 10, the user device 20) may generate integrated time-series data based on images captured by the visible ray camera, and may estimate biometric information according to the operation method of FIG. 5 described above. For example, unlike described above, the electronic device (for example, the server 10, the user device 20) may generate integrated time-series data based on images captured by the IR camera, and may estimate biometric information according to the operation method of FIG. 9.

According to various embodiments, when information on the photographing condition is a first range, operations based on the RGB camera may be performed, and, when the information on the photographing condition is a second range lower than the first range, operations based on the IR camera may be performed, and, when the information on the photographing condition is a third range between the first range and the second range, hybrid operations based on the RGB camera and the IR camera may be performed. However, this should not be considered as limiting. For example, when illuminance is a first range, operations based on the RGB camera may be performed, when the illuminance is a second range lower than the first range, operations based on the IR camera may be performed, and, when the illuminance is a third range between the first range and the second range, hybrid operations may be performed.

### 3.4 AI model-based rPPG/biometric information measurement operation

### 3.4.1 Blood pressure AI model-based blood pressure measurement

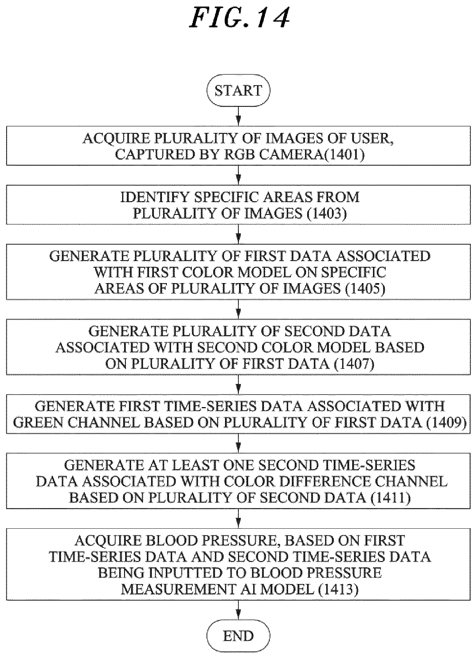

FIG. 14 is a flowchart provided to explain an example of an operation method of an electronic device (for example, the server 10, the user device 20) for measuring a blood pressure based on a blood pressure AI model according to various embodiments. However, operations may be performed in order different from the order of operations described and/or illustrated, and more operations or fewer operations than the operations described and/or illustrated may be performed.

According to various embodiments, the electronic device (for example, the server 10, the user device 20) may acquire a plurality of images of a user, captured by an RGB camera in operation 1401, may identify specific areas from the plurality of images in operation 1403, and may generate a plurality of first data associated with a first color model on the specific areas of the plurality of images in operation 1405. For example, the electronic device (for example, the server 10, the user device 20) may acquire RGB color model values on a skin area R2 on the plurality of captured images for a pre-set time as described in operations 501 to 505.

According to various embodiments, the electronic device (for example, the server 10, the user device 20) may generate a plurality of second data associated with a second color model based on the plurality of first data in operation 1407, may generate first time-series data associated with a green channel based on the plurality of first data in operation 1409, and may generate at least one second time-series data associated with a color difference channel based on the plurality of second data in operation 1411. For example, the electronic device (for example, the server 10, the user device 20) may perform an operation of generating time-series data (rPPG) of each of the plurality of color models (for example, RGB, YCgCr, CIE La*b*) in operations 1407 to 1411 as described above.

According to various embodiments, the electronic device (for example, the server 10, the user device 20) may acquire a blood pressure based on the first time-series data and the second time-series data being inputted to a blood pressure measurement AI model in operation 1413. For example, the blood pressure measurement AI model may be an AI model that is trained to output a blood pressure value based on information on at least a part of time-series data among the plurality of color models (for example, RGB, YCgCr, CIE La*b*) being inputted. The training may be performed based on various learning algorithms such as supervised learning, non-supervised learning, machine learning, etc., and thus a detailed description is omitted.

In an embodiment, the blood pressure AI model may be an AI model that is trained as at least a part of time-series data among the plurality of color models (for example, RGB, YCgCr, CIE La*b*) of a specific time period is set as input data, and a blood pressure measured for a specific time is set as output data.

In an embodiment, the blood pressure measurement AI model may be an AI model that is trained as a frequency characteristic value of at least a part of the plurality of color models (for example, RGB, YCrCr, CIE La*b*) of a specific time period is set as input data, and a blood pressure value measured for a specific time is set as output data. The frequency characteristic value may include information on an inter-peak distance.

### 3.4.2 Stress analysis AI model-based stress measurement

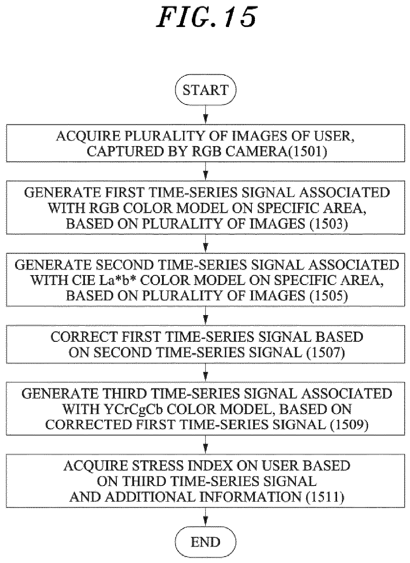

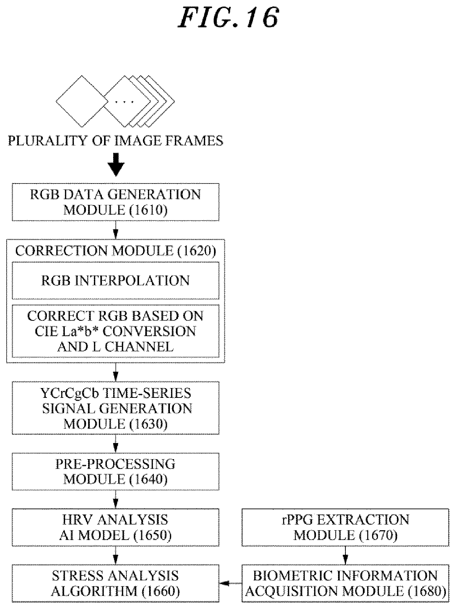

FIG. 15 is a flowchart provided to explain an example of an operation method of an electronic device (for example, the server 10, the user device 20) for measuring stress based on a stress analysis AI model. However, operations may be performed in order different from the order of operations described and/or illustrated, and more operations or fewer operations than the operations described and/or illustrated may be performed. Hereinbelow, FIG. 15 will be described in detail with reference to FIG. 16.

FIG. 16 is a view provided to explain examples of modules for performing stress analysis operations according to various embodiments.

According to various embodiments, the electronic device (for example, the server 10, the user device 20) may acquire a plurality of images of a user, captured by an RGB camera in operation 1501, and may generate a first time-series signal (for example, an RGB color model value) associated with an RGB color model on the specific area (for example, the skin area R2 of FIG. 5) based on the plurality of images in operation 1503. Operations 1501 to 1503 of the electronic device (for example, the server 10, the user device 20) may be performed based on an RGB generation data module 1610 like operations 501 to 503 of the electronic device (for example, the server 10, the user device 20) described above, and a redundant explanation is omitted.

According to various embodiments, the electronic device (for example, the server 10, the user device 20) may generate a second time-series signal associated with a CIE La*b* color model on the specific area based on the plurality of images in operation 1505, and may correct the first time-series signal based on the second time-series signal in operation 1507. For example, referring to FIG. 16, the electronic device (for example, the server 10, the user device 20) may interpolate the first time-series signal associated with the RGB color model on the skin area R2 by using a correction module 1620, and may correct (or pre-process) brightness of the first time-series signal by using the second time-series signal on an L channel which is measured from the plurality of images converted into the CIE La*b* color model.

According to various embodiments, the electronic device (for example, the server 10, the user device 20) may generate a third time-series signal associated with the YCrCgCb color model based on the corrected first time-series signal in operation 1509. The electronic device (for example, the server 10, the user device 20) may generate a YCrCgCb time-series signal based on the first time-series signal on the RGB model, the brightness of which is pre-processed, by using a YCrCgCb time-series signal generation module 1630, and may pre-process the YCrCgCb time-series signal by using a pre-processing model 1640. The pre-processing operation may include at least one of moving average filter-based noise removal, sliding window-based detrending, butterworth bandpass filtering, amplitude correction, or extracting (or cropping) a signal of a pre-set time (for example, 8 seconds) with reference to down peak after detecting signal peak of each channel (Y, Cr, Cg, Cb).

According to various embodiments, the electronic device (for example, the server 10, the user device 20) may acquire a stress index on the user, based on the third time-series signal and additional information in operation 1511. For example, the electronic device (for example, the server 10, the user device 20) may acquire a blood pressure feature value (for example, SDNN, SDSD, RMSSD) extracted based on the pre-processed third time-series signal (YCrCgCb signal) being inputted to a heart rate variability (HRV) analysis AI model 1650. The electronic device (for example, the server 10, the user device 20) may acquire a stress index based on rPPG acquired based on the rPPG extraction module and biometric information (for example, a pulse rate, oxygen saturation) measured by rPPG based on a biometric information acquisition module 1680 being inputted to a stress index analysis algorithm (equation), along with the acquired blood pressure feature value. The measured rPPG and biometric information may be measured as described above in FIGS. 5, 9, and 12, and a redundant explanation is omitted. The electronic device (for example, the server 10, the user device 20) may perform post-processing with respect to the acquired stress index based on a post-processing algorithm, and may provide the stress index to the user.

## 4. Mobility service

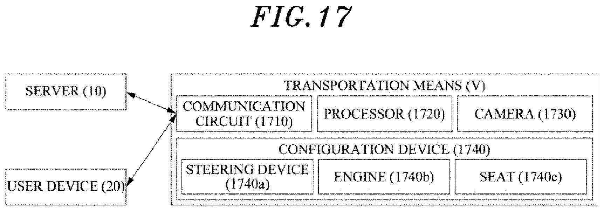

FIG. 17 is a view provided to explain an example of a transportation means V for providing a service based on non-contact biometric information according to various embodiments.

According to various embodiments, an electronic device (for example, the server 10, the user device 20) may be implemented to measure rPPG and biometric information based on analysis of a captured image for a passenger (for example, a driver or a passenger) riding in the transportation means V, and to provide a predetermined service according to the rPPG and biometric information measurement method described in "Content 3" above.

According to various embodiments, referring to FIG. 17, the transportation means V may include a communication circuit 1710, a processor 1720, a camera 1730, and a configuration device 1740, but is not limited to the example illustrated and may be implemented to include more devices. The camera 1730 may not be provided in the transportation means V and may perform a service based on a captured image by the camera 430 of the user device 20 held in the transportation means V.

According to various embodiments, the electronic device (for example, the server 10, the user device 20) may receive various types of information from the communication circuit 1710 of the transportation means V. For example, the various types of information may include an image of a passenger (for example, a driver or a passenger) riding in the transportation means V, which is captured by the camera 1730, and information on a photographing environment condition.

According to various embodiments, the electronic device (for example, the server 10, the user device 20) may transmit a control signal for causing the processor 1720 of the transportation means V to control the configuration device 1740 based on rPPG and biometric information analyzed as a result to the transportation means V. The processor 1720 of the transportation means V may control the configuration device 1740 based on the received control signal. For example, the configuration device 1740 may include not only a device that directly influences driving such as a steering device 1740a and an engine 1740b, but also a device that does not directly influence driving but provides convenience such as a seat 1740c, a light (not shown), etc.

## 4.1 Providing mobility service based on non-contact biometric information and outside information

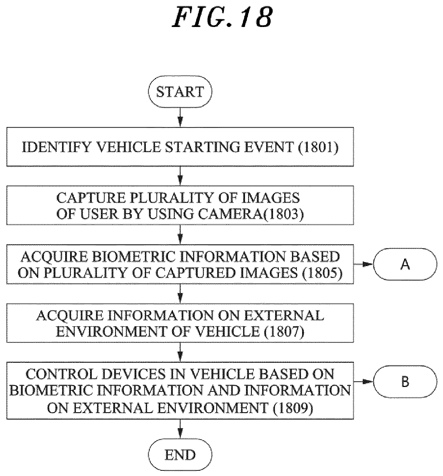

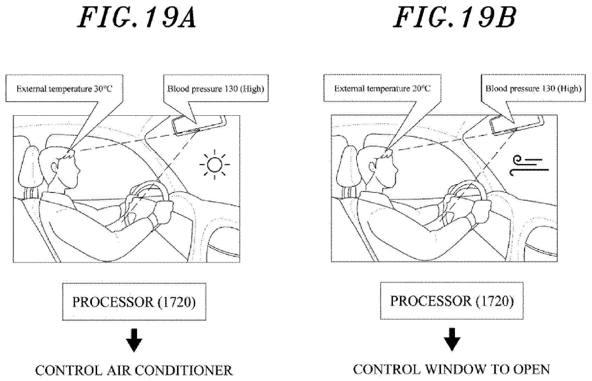

FIG. 18 is a flowchart provided to explain an example of an operation method of an electronic device (for example, the server 10, the user device 20) for providing a mobility service based on non-contact biometric information and outside information. However, operations may be performed in order different from the order of operations described and/or illustrated, and more operations or fewer operations than the operations described and/or illustrated may be performed. Hereinbelow, FIG. 18 will be described in detail with reference to FIGS. 19A and 19B.

FIGS. 19A and 19B are a view provided to explain an example of a mobility service which is provided according to non-contact biometric information and outside information according to various embodiments.

According to various embodiments, the electronic device (for example, the server 10, the user device 20) may identify a vehicle starting event in operation 1801, may capture a plurality of images of a user, captured by a camera in operation 1803, and may acquire biometric information based on the plurality of captured images in operation 1805. For example, the electronic device (for example, the server 10, the user device 20) may acquire a plurality of images by using the camera 1730 provided in the transportation means (for example, a vehicle) or the camera 430 provided in the user device 20 held in the transportation means (for example, a vehicle), and may acquire biometric information. The camera 1730, 430 may include at least one of a visible ray camera or an IR camera, and the operation of measuring rPPG and biometric information may be performed as described above in "Content 3" and a redundant explanation is omitted.

According to various embodiments, the electronic device (for example, the server 10, the user device 20) may acquire information on an external environment of the vehicle in operation 1807, and may control devices in the vehicle based on the biometric information and the information on the external environment in operation 1809. For example, the electronic device (for example, the server 10, the user device 20) may acquire information on the external environment associated with a location of the vehicle which is currently started. The information on the external environment may include information on a climate such as temperature, wind direction, wind speed, weather, etc. of the location of the vehicle, and various types of information collectable from an external server. As a result, the electronic device (for example, the server 10, the user device 20) may provide the vehicle with a control signal for controlling the configuration device 1740 of the vehicle based on the biometric information measured in a non-contact method and the information on the external environment collected. For example, referring to FIGS. 19A and 19B, the electronic device (for example, the server 10, the user device 20) may provide control signals for controlling different types of configuration devices according to a blood pressure measured in the non-contact method and an external temperature measured as external environment information. Referring to FIGS. 19A and 19B, when a blood pressure value is a specific value (for example, 130), the electronic device (for example, the server 10, the user device 20) may determine that an event for controlling the configuration device 1740 of the vehicle occurs. When the event occurs, the electronic device (for example, the server 10, the user device 20) may acquire information on an external temperature as external environment information, and, when the external temperature is higher than a pre-set value as shown in FIG. 19A, the electronic device (for example, the server 10, the user device 20) may generate a control signal for activating an air conditioner among the configuration devices 1740, and, when the external temperature is lower than the pre-set value as shown in FIG. 19B, the electronic device (for example, the server 10, the user device 20) may generate a control signal for opening a window among the configuration devices 1740, and may provide the control signal to the processor 1720 of the vehicle. The processor 1720 of the vehicle may control the configuration device 1740 of the vehicle based on the control signal. The disclosure is not limited to the example described and/or illustrated, and various types of operations of controlling the configuration device 1740 of the vehicle may be performed, and operations for controlling other configuration devices 1740 may be performed in a similar method and a detailed description is omitted.

According to various embodiments, as a part of the operations of generating the control signal described above, the electronic device (for example, the server 10, the user device 20) may use at least one AI model that stores information on types of the configuration devices 1740 to be controlled and control methods according to biometric information and information on the external environment in the form of a look-up table, or pre-learns information on types of the configuration devices 1740 to be controlled and control methods according to biometric information and information on the external environment.

**4.1.1 Providing a mobility service further considering passenger information**

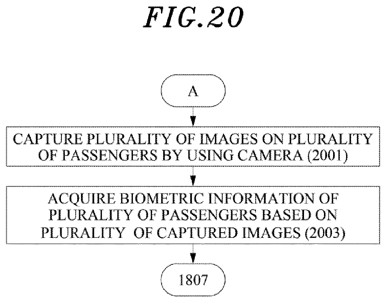

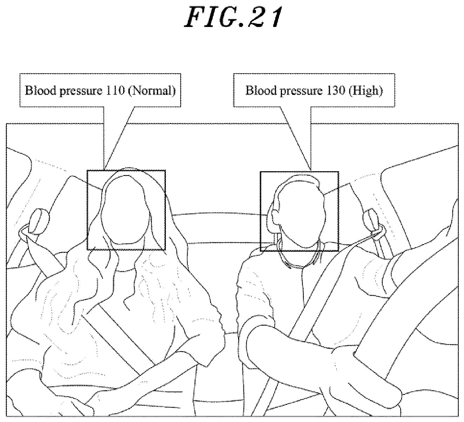

FIG. 20 is a flowchart provided to explain an example of an operation method of an electronic device (for example, the server 10, the user device 20) for providing a mobility service further considering passenger information. However, operations may be performed in order different from the order of operations described and/or illustrated, and more operations or fewer operations than the operations described and/or illustrated may be performed. Hereinbelow, FIG. 20 will be described in detail with reference to FIG. 21.

FIG. 21 is a view provided to explain an example of measurement of rPPG/biometric information on a passenger according to various embodiments.

According to various embodiments, the electronic device (for example, the server 10, the user device 20) may provide a mobility service further considering biometric information on a passenger in the vehicle when providing the mobility service as described in "Content 4.1".

According to various embodiments, the electronic device (for example, the server 10, the user device 20) may capture a plurality of images for a plurality of passengers by using a camera in operation 2001, may acquire biometric information of the plurality of passengers based on the plurality of captured images in operation 2003, and may provide a mobility service by performing operations 1807 to 1809 as a result. For example, referring to FIG. 21, not only a driver but also a passenger may ride in the vehicle, and in the case of an autonomous vehicle, a plurality of passengers may ride. In this case, when a mobility service is provided considering only biometric information of the driver, inconvenience may be caused to other passengers, and therefore, the electronic device (for example, the server 10, the user device 20) may further consider biometric information of the other passengers when providing the mobility service.

For example, the electronic device (for example, the server 10, the user device 20) may acquire a plurality of captured images for the plurality of passengers for a pre-set time by using the camera 1730 provided in the transportation means (for example, a vehicle) or the camera 430 provided in the user device 20 held in the transportation means (for example, a vehicle). In an embodiment, the camera 1730 may be designed to have a field of view (FOV) to be able to capture all of the plurality of passengers or may be provided at a location (for example, a room mirror, a ceiling) to be able to capture all of the plurality of passengers. Alternatively, a plurality of cameras 1730 may be provided at loations adjacent to the seats of the plurality of passengers. In an embodiment, the camera 430 may be designed to have a FOV to be able to capture all of the plurality of passengers, or the user devices 20 that the plurality of passengers carry may be held at locations adjacent to the respective seats, such that images for the passengers may be captured.

Accordingly, the electronic device (for example, the server 10, the user device 20) may generate different control signals by further considering biometric image measured based on the images of the passengers. For example, when the biometric information of the plurality of passengers is included in a similar range, the electronic device (for example, the server 10, the user device 20) may generate the determined control signal of the configuration device 1740 as it is. For example, when the biometric information of the plurality of passengers is not included in the similar range (that is, is different) as shown in FIG. 21, the electronic device (for example, the server 10, the user device 20) may generate a control signal for opening a window, and specifically, may generate a control signal for opening only the window near to the driver for the driver who has a high pressure blood, and for keeping the window near to the passenger who is in a normal range of blood pressure as it is.

**4.1.2 Providing a mobility service further considering a mode of a vehicle.**

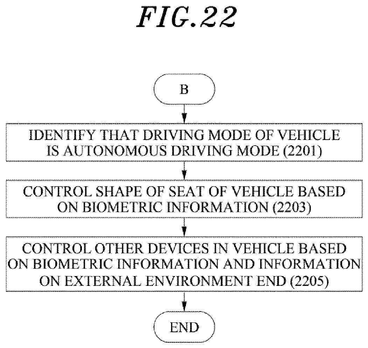

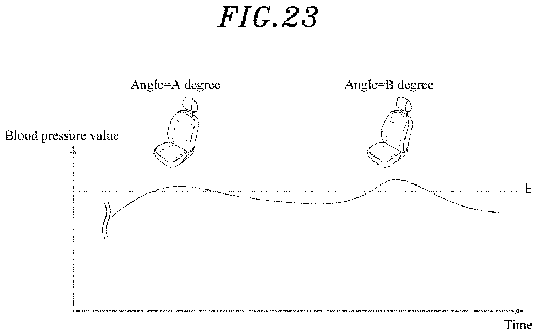

FIG. 22 is a flowchart provided to explain an example of an operation method of an electronic device (for example, the server 10, the user device 20) for providing a mobility service further considering a mode of a vehicle. However, operations may be performed in order different from the order of operations described and/or illustrated, and more operations or fewer operations than the operations described and/or illustrated may be performed. Hereinbelow, FIG. 22 will be described in detail with reference to FIG. 23.

FIG. 23 is a view provided to explain an example of a mobility service which deforms a configuration of a seat continuously during autonomous driving according to various embodiments.

According to various embodiments, the electronic device (for example, the server 10, the user device 20) may identify that a driving mode of the vehicle is an autonomous driving mode in operation 2201, and may control the configuration of a seat of the vehicle based on the biometric information in operation 2203. For example, the vehicle may be set to an autonomous driving mode under control of the driver after being started. As shown in FIG. 23, the electronic device (for example, the server 10, the user device 20) may continuously (or periodically) measure biometric information (for example, a blood pressure) of the user based on captured images in the autonomous driving mode, and may control the configuration of the seat of the vehicle to change to a configuration comfortable for the user based on the biometric information measured during the autonomous driving mode, so that user's convenience can be further enhanced. For example, when it is identified that a blood pressure value measured in a non-contact method exceeds a pre-set value E as shown in FIG. 23, the electronic device (for example, the server 10, the user device 20) may generate a control signal for changing the angle of the seat from a current angle to a different angle and may provide the control signal to the processor 1820 of the vehicle.

According to various embodiments, the electronic device (for example, the server 10, the user device 20) may control other devices in the vehicle based on the biometric information and information on an external environment in operation 2205. Operation 2205 of the electronic device (for example, the server 10, the user device 20) may be performed in the same way as operation 1908 of the electronic device (for example, the server 10, the user device 20) described above, and a redundant explanation thereof is omitted.

### 5. Providing continuous biometric information accumulation-based service

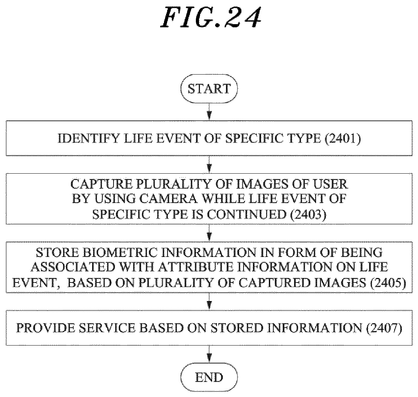

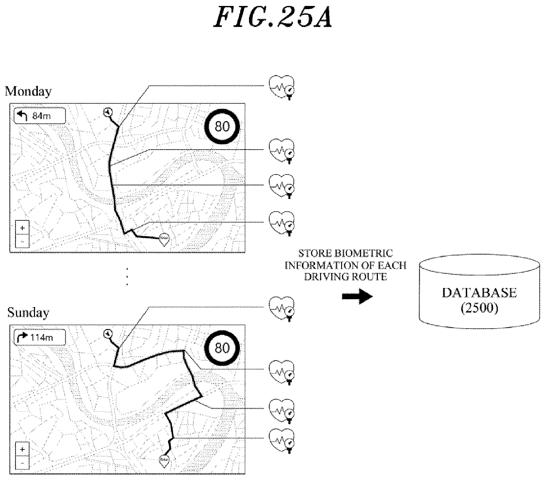

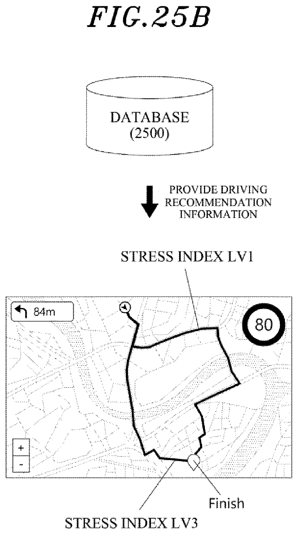

FIG. 24 is a flowchart provided to explain an example of an operation method of an electronic device (for example, the server 10, the user device 20) for providing a continuous biometric information accumulation-based service. However, operations may be performed in order different from the order of operations described and/or illustrated, and more operations or fewer operations than the operations described and/or illustrated may be performed. Hereinbelow, FIG. 24 will be described in detail with reference to FIGS. 25A and 25B.

FIG. 25A is a view provided to explain an embodiment in which biometric information measured by driving routes for a pre-set time is accumulated in a database 2500 according to various embodiments. FIG. 25B is a view provided to explain an example of a service which provides driving recommendation information based on accumulated biometric information according to various embodiments.

According to various embodiments, the electronic device (for example, the server 10, the user device 20) may identify a life event of a specific type in operation 2401, and may capture a plurality of images of a user, captured by a camera while the life event of the specific type is continued. For example, the life event of the specific type may refer to various activities that the user may perform, and for example, may include various types of activities such as driving, exercising, studying, working life, etc. In this case, the electronic device (for example, the server 10, the user device 20) may receive an input from the user by providing a graphic user interface for receiving an input of information indicating what life event occurs, and may predict a type of a life event that the user is currently doing, based on pattern information of a sensor value collected by using various types of sensors (for example, a motion sensor, an angular speed sensor, an illuminance sensor, etc.). When the life event of the specific type is performed, the electronic device (for example, the server 10, the user device 20) may capture images of the user at a plurality of times periodically based on the camera 430 of the user device 20 or a camera (for example, the camera 1730 in the vehicle) disposed in a space where the life event of the specific type is performed, and may measure rPPG and biometric information of the user at the plurality of times based on the captured images as described in "Content 3". For example, when the user drives along a specific route on each of the plurality of days for a pre-set period as shown in FIG. 25A, biometric information (for example, a blood pressure, stress) obtained during the driving along the corresponding driving route may be stored based on captured images by the camera 1730 provided in the vehicle or the camera 430 of the user device held in the vehicle at each time. The stored biometric information may include at least one of biometric information of each time of the driving route or average biometric information. The disclosure is not limited to the example described and/or illustrated and it is obvious to those skilled in the art that other types of life events (for example, exercising, studying, working life, etc.) and/or various types of biometric information (for example, pulse rate, SPO2, etc.)

According to various embodiments, the electronic device (for example, the server 10, the user device 20) may store the biometric information in the form of being associated with attribute information on the life event based on the plurality of captured images in operation 2405. For example, as shown in FIG. 25A, the electronic device (for example, the server 10, the user device 20) may accumulate blood pressure information by different driving routes in a life event category named "user's driving". That is, the attribute information may refer to an element that defines the same life event category, and in the case of driving, the attribute information may be information on driving routes (for example, a point of departure, a point of arrival, a route), a driving time. In the case of exercising, information like types of exercise, time of exercise may be attribute information on the exercise life vent.

According to various embodiments, the electronic device (for example, the server 10, the user device 20) may provide a service based on the stored information in operation 2407. For example, the electronic device (for example, the server 10, the user device 20) may provide accumulation information of each life category of the user which is stored for a pre-set period to another external server, or may provide a service by itself based on pre-set accumulation information. The accumulated information may be personalized information only for the user, and may be used as an objective basis to provide an optimized service to the user according to a life category. For example, when a route to drive from a first location to a second location is recommended, the electronic device (for example, the server 10, the user device 20) may provide information on a stress index for each driving route to drive from the first location to the second location, based on biometric information (for example, a stress index) of each driving route from the first location to the second location, which is accumulated in the database 2500 as shown in FIG. 25B. Accordingly, the user may select a driving route according to a driving time and a driving distance, and may also select a driving route by considering the stress index (or change of the biometric information) that the user may experience.

According to various embodiments, the electronic device (for example, the server 10, the user device 20) may determine whether to provide a service based on the stored information, based on current biometric information of the user. For example, when a value of rPPG and/or biometric information currently measured in a non-contact method satisfies a pre-set condition, the electronic device (for example, the server 10, the user device 20) may initiate the operation of providing the service based on the stored information. For example, when at least one of the blood pressure or the stress index is greater than or equal to a pre-set value, the need for reference to biometric information during user's life may increase, and accordingly, the electronic device (for example, the server 10, the user device 20) may perform the operation of providing the service based on the biometric information stored according to each life category. In other words, when the need for reference to biometric information during user's life is low, a normal service may be performed.

### 6. Kiosk service

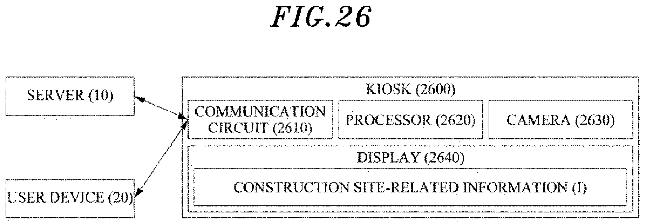

FIG. 26 is a view provided to explain an example of a kiosk 2600 for providing a service based on non-contact biometric information according to various embodiments.

According to various embodiments, the electronic device (for example, the server 10, the user device 20) may be implemented to measure rPPG and biometric information based on analysis of an image of a user captured by the kiosk 2600 disposed in various places in the same way as the rPPG and biometric measurement method described in "Content 3", and to provide a predetermined service. The kiosk 2600 may be placed in a place where workers move, and may be placed in various places such as a doorway, an aisle, etc.

According to various embodiments, referring to FIG. 26, the kiosk 2600 may include a communication circuit 2610, a processor 2620, a camera 2630, and a display 2640, and is not limited to the example illustrated and may be implemented to include more devices.

According to various embodiments, the electronic device (for example, the server 10, the user device 20) may receive various types of information from the communication circuit 2610 of the kiosk 2600. For example, the various types of information may include an image of a worker captured by the camera 2630 and information on a photographing environment condition.

According to various embodiments, the electronic device (for example, the server 10, the user device 20) may transmit a control signal for causing the processor 2620 of the kiosk 2600 to output predetermined information (for example, construction site-related information (I)) through the display 2640, based on rPPG and biometric information analyzed as a result. In this case, the construction site-related information (I) displayed through the display 2640 of the kiosk 2600 may include not only information received from the electronic device (for example, the server 10, the user device 20) but also information (for example, weather information) received from other external servers.

### 6.1 Providing a service based on biometric information of a plurality of locations

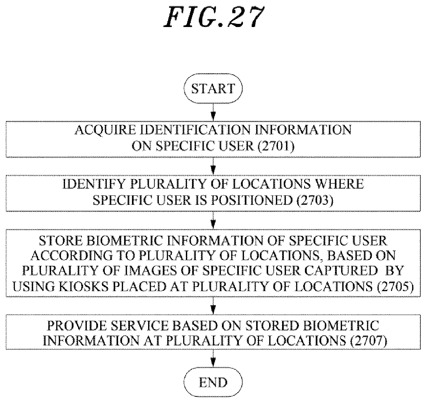

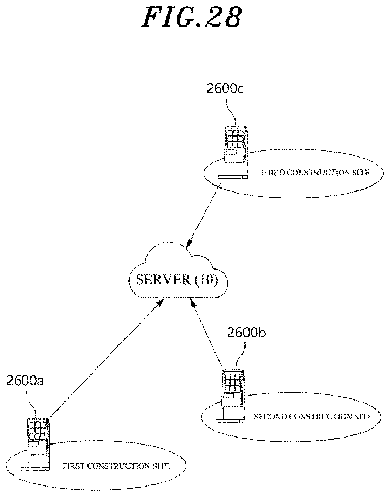

FIG. 27 is a flowchart provided to explain an example of an operation method of an electronic device (for example, the server 10, the user device 20) for providing a service based on biometric information of a plurality of locations. However, operations may be performed in order different from the order of operations described and/or illustrated, and more operations or fewer operations than the operations described and/or illustrated may be performed. Hereinbelow, FIG. 27 will be described in detail with reference to FIGS. 28 to 29.

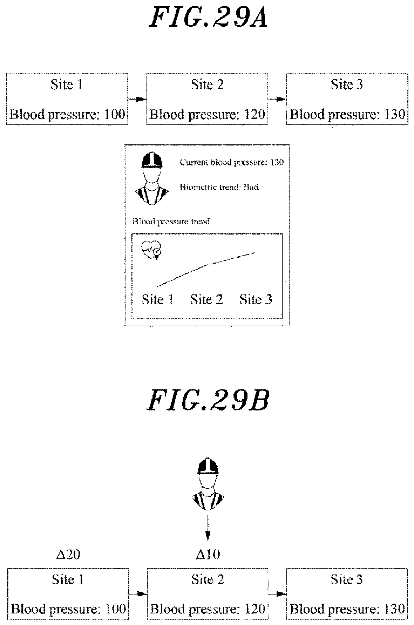

FIG. 28 is a view provided to explain an example of collecting biometric information on a specific user from a kiosk disposed at each of a plurality of locations according to various embodiments. FIGS. 29A and 29B are a view provided to explain an example of a service based on biometric information at a plurality of locations according to various embodiments.

According to various embodiments, the electronic device (for example, the server 10, the user device 20) may acquire identification information on a specific user in operation 2701, may identify a plurality of locations where the specific user is positioned in operation 2703, and may store biometric information of the specific user at the plurality of location, based on a plurality of images of the specific user which are captured by using kiosks placed at the plurality of locations in operation 2705. For example, referring to FIGS. 29A and 29B, biometric information on the specific user (or worker) may be acquired based on the kiosks 2600 disposed at the plurality of construction sites. For example, when the specific user enters a first construction site at a first time, the first kiosk 2600a may provide a captured image for the specific user to the electronic device (for example, the server 10, the user device 20) along with reference information (for example, time, region). For example, when the specific user enters another construction site (for example, a second construction site, a third construction site) at a different time after the first time, the electronic device (for example, the server 10, the user device 20) may receive an image on the specific user which is captured by the kiosks 2600b, 2600c placed in other construction sites (for example, the second construction site, the third construction site) along with reference information (for example, time, region). The electronic device (for example, the server 10, the user device 20) may identify the specific user based on a facial image included in the captured image and a pre-registered facial image, and may store biometric information measured based on the captured image according to a location of the identified specific user, based on reference information. The operation of measuring the biometric information based on the captured image may be performed in the same way as described above in "Content 3", and a redundant explanation is omitted.

According to various embodiments, the electronic device (for example, the server 10, the user device 20) may provide a service based on the biometric information at the plurality of locations in operation 2707. For example, as shown in FIG. 29A, the electronic device (for example, the server 10, the user device 20) may provide trend information on the biometric information (for example, a blood pressure) in the respective construction sites where the specific user is positioned in sequence through the kiosk 2600. The electronic device (for example, the server 10, the user device 20) may estimate biometric information that will increase in other construction sites where the specific user will work next time, based on trend information of the biometric information, and, when the estimated biometric information exceeds a pre-set value, the electronic device (for example, the server 10, the user device 20) may prevent the specific user from entering the other construction sites, thereby preventing an accident. For example, as shown in FIG. 29B, the electronic device (for example, the server 10, the user device 20) may select a location (for example, a workplace) appropriate for the user, based on the biometric information which increases by locations, and may provide information thereon, thereby allowing the user to be deployed in a workplace where the user can work more smoothly. For example, the workplace where the user's blood pressure is lowest may be identified as the most appropriate workplace to the user. When there are a plurality of workers and the user's blood pressure is lower than the average blood pressure of the plurality of workers, the corresponding workplace may be determined to be appropriate.

(1) An operation method of an electronic device comprises: acquiring a plurality of images of a user, captured by an RGB camera of the electronic device; acquiring a specific area on a specific body part of the user from the plurality of images; generating a plurality of first data associated with an RGB color model on the specific area associated with the plurality of images; generating a plurality of second data associated with a YCrCb color model based on the plurality of first data; generating first time-series data associated with a green channel based on the plurality of first data; generating second time-series data associated with a color difference channel based on the plurality of second data; generating third time-series data based on combining the first time-series data and the second time-series data; and estimating a pulse rate of the user based on conversion of the third time-series data into a frequency domain.

(2) The method according to (1) may have the acquiring the specific area on the specific body part of the user from the plurality of images including: identifying values on a specific color model on the plurality of images; and extracting an area corresponding to a range of a pre-set value among the identified values.

(3) The method according to (1) or (2) may have the generating the plurality of first data associated with the RGB color model including: acquiring a plurality of values for each of a plurality of color channels of the RGB color model for each of the plurality of images, each of the plurality of values corresponding to a specific time point; identifying a plurality of interval values of a plurality of time windows among the plurality of values of the plurality of color channels; and generating the plurality of first data by adjusting the plurality of values based on an average of the plurality of interval values.

(4) The method according to any one of (1) to (3) may have performing at least one of a noise removal operation by using a signal filter or a trend correction operation with respect to the plurality of first data.

(5) The method according to any one of (1) to (4) may have the generating the first time-series data associated with the green channel including: generating first sub time-series data by subtracting a value of a red channel from a value of the green channel, and generating second sub time-series data by subtracting a value of a blue channel from the value of the green channel; and generating the first time-series data by combining the first sub time-series data and the second sub time-series data.

(6) The method according to (5) may have generating the plurality of second data for the YCrCb color model from the plurality of first data; and generating the second time-series data by combining third sub time-series data of a Cr channel and fourth sub time-series data of a Cb channel based on the plurality of second data.

(7) The method according to any one of (1) to (6) may have generating a plurality of third data for a CIE La*b* color model from the plurality of first data; generating third time-series data based on fifth sub time-series data of *a* channel based on the plurality of third data; and wherein the generating integrated time-series data comprises: generating the integrated time-series data by combining the first time-series data, the second time-series data, and the third time-series data.

(8) A non-transitory computer-readable storage medium having a program recorded thereon for executing the method according to any one of (1) to (7).

(9) An electronic device comprising at least one processor, wherein the at least one processor is configured to: acquire a plurality of images of a user, captured by an RGB camera of the electronic device;

acquire a specific area on a specific body part of the user from the plurality of images; generate a plurality of first data associated with an RGB color model on the specific area associated with the plurality of images; generate a plurality of second data associated with a YCrCb color model based on the plurality of first data; generate first time-series data associated with a green channel based on the plurality of first data; generate second time-series data associated with a color difference channel based on the plurality of second data; generate integrated time-series data based on combining the first time-series data and the second time-series data; and estimate a pulse rate of the user by converting the integrated time-series data into a frequency domain.

(10) The electronic device according to (9) may have the at least one processor which is configured to, as at least a part of the operation of acquiring the specific area on the specific body part of the user from the plurality of images: identify values on a specific color model on the plurality of images; and extract an area corresponding to a range of a pre-set value among the identified values.

(11) The electronic device according to (9) or (10) may have the at least one processor which is configured to, as at least a part of the operation of generating the plurality of first data associated with the RGB color model: acquire a plurality of values for each of a plurality of color channels of the RGB color model for each of the plurality of images, each of the plurality of values corresponding to a specific time point; identify a plurality of interval values of a plurality of time windows among the plurality of values of the plurality of color channels; and generate the plurality of first data by adjusting the plurality of values based on an average of the plurality of interval values.

(12) The electronic device according to any one of (9) to (11) may have the at least one processor which is further configured to perform at least one of a noise removal operation or a trend correction operation with respect to the plurality of first data.

(13) The electronic device according to any one of (9) to (12) may have the at least one processor is configured to: generate first sub time-series data by subtracting a value of a red channel from a value of the green channel, and generate second sub time-series data by subtracting a value of a blue channel from the value of the green channel; and generate the first time-series data by combining the first sub time-series data and the second sub time-series data.

(14) The electronic device according to (13) may have the at least one processor which is configured to: generate the plurality of second data for the YCrCb color model from the plurality of first data; and generate the second time-series data by combining third sub time-series data of a Cr channel and fourth sub time-series data of a Cb channel based on the plurality of second data.

(15) The electronic device according to any one of (9) to (14) may have the at least one processor which is further configured to: generate a plurality of third data for a CIE La*b* color model from the plurality of first data; and generate third time-series data based on fifth sub time-series data of $a$ channel based on the plurality of third data; and wherein the at least one processor is configured to, as at least a part of the operation of generating the integrated time-series data: generate the integrated time-series data by combining the first time-series data, the second time-series data, and the third time-series data.

**Claims**

1. An operation method of an electronic device (20) comprising:

    acquiring (501) a plurality of images of a user, captured by an RGB camera (430) of the electronic device (20);
    acquiring (503) a specific area on a specific body part of the user from the plurality of images;
    generating (505) a plurality of first data associated with an RGB color model on the specific area associated with the plurality of images;
    generating (507) a plurality of second data associated with a YCrCb color model based on the plurality of first data;
    generating (509) first time-series data associated with a green channel based on the plurality of first data;
    generating (511) second time-series data associated with a color difference channel based on the plurality of second data;
    generating (513) integrated time-series data based on combining the first time-series data and the second time-series data; and
    estimating (515) a pulse rate of the user by converting the integrated time-series data into a frequency domain.

2. The operation method of claim 1, wherein the acquiring the specific area on the specific body part of the user from the plurality of images comprises:

    identifying values on a specific color model on the plurality of images; and
    extracting an area corresponding to a range of a pre-set value among the identified values.

3. The operation method of claim 1 or 2, wherein the generating the plurality of first data associated with the RGB color model comprises:

acquiring a plurality of values for each of a plurality of color channels of the RGB color model for each of the plurality of images, each of the plurality of values corresponding to a specific time point; identifying a plurality of interval values of a plurality of time windows among the plurality of values of the plurality of color channels; and generating the plurality of first data by adjusting the plurality of values based on an average of the plurality of interval values.

4. The operation method of any one of claims 1 to 3, further comprising performing at least one of a noise removal operation by using a signal filter or a trend correction operation with respect to the plurality of first data.

5. The operation method of any one of claims 1 to 4, wherein the generating the first time-series data associated with the green channel comprises:

generating first sub time-series data by subtracting a value of a red channel from a value of the green channel, and generating second sub time-series data by subtracting a value of a blue channel from the value of the green channel; and generating the first time-series data by combining the first sub time-series data and the second sub time-series data.

6. The operation method of claim 5, further comprising:

generating the plurality of second data for the YCrCb color model from the plurality of first data; and generating the second time-series data by combining third sub time-series data of a Cr channel and fourth sub time-series data of a Cb channel based on the plurality of second data.

7. The operation method of any one of claims 1 to 6, further comprising:

generating a plurality of third data for a CIE La*b* color model from the plurality of first data; generating third time-series data based on fifth sub time-series data of a channel based on the plurality of third data; and wherein the generating integrated time-series data comprises: generating the integrated time-series data by combining the first time-series data, the second time-series data, and the third time-series data.

8. A non-transitory computer-readable storage medium having a program recorded thereon for executing the method according to any one of claims 1 to 7.

9. An electronic device (20) comprising at least one processor (410), wherein the at least one processor (410) is configured to:

acquire a plurality of images of a user, captured by an RGB camera (430) of the electronic device (20); acquire a specific area on a specific body part of the user from the plurality of images; generate a plurality of first data associated with an RGB color model on the specific area associated with the plurality of images; generate a plurality of second data associated with a YCrCb color model based on the plurality of first data; generate first time-series data associated with a green channel based on the plurality of first data; generate second time-series data associated with a color difference channel based on the plurality of second data; generate integrated time-series data based on combining the first time-series data and the second time-series data; and estimate a pulse rate of the user by converting the integrated time-series data into a frequency domain.

10. The electronic device of claim 9, wherein the at least one processor (410) is configured to, as at least a part of the operation of acquiring the specific area on the specific body part of the user from the plurality of images:

identify values on a specific color model on the plurality of images; and extract an area corresponding to a range of a pre-set value among the identified values.

11. The electronic device of claim 9 or 10, wherein the at least one processor (410) is configured to, as at least a part of the operation of generating the plurality of first data associated with the RGB color model:

acquire a plurality of values for each of a plurality of color channels of the RGB color model for each of the plurality of images, each of the plurality of values corresponding to a specific time point; identify a plurality of interval values of a plurality of time windows among the plurality of values of the plurality of color channels; and generate the plurality of first data by adjusting the plurality of values based on an average of the plurality of interval values.

12. The electronic device of any one of claims 9 to 11, wherein the at least one processor (410) is further configured to perform at least one of a noise removal operation or a trend correction operation with respect to the plurality of first data.

13. The electronic device of any one of claims 9 to 12, wherein the at least one processor (410) is configured to:

generate first sub time-series data by subtracting a value of a red channel from a value of the green channel, and generate second sub time-series data by subtracting a value of a blue channel from the value of the green channel; and generate the first time-series data by combining the first sub time-series data and the second sub time-series data.

14. The electronic device of claim 13, wherein the at least one processor (410) is configured to:

generate the plurality of second data for the YCrCb color model from the plurality of first data; and generate the second time-series data by combining third sub time-series data of a Cr channel and fourth sub time-series data of a Cb channel based on the plurality of second data.

15. The electronic device of any one of claims 9 to 14, wherein the at least one processor (410) is further configured to:

generate a plurality of third data for a CIE La*b* color model from the plurality of first data; and generate third time-series data based on fifth sub time-series data of *a* channel based on the plurality of third data; and wherein the at least one processor is configured to, as at least a part of the operation of generating the integrated time-series data: generate the integrated time-series data by combining the first time-series data, the second time-series data, and the third time-series data.

# FIG.1

# FIG.2

## FIG.3

| SERVER (10) |
|---|
| FIRST PROCESSOR (210) |
| FIRST COMMUNICATION CIRCUIT (220) |
| FIRST MEMORY (230) |

## FIG.4

| ELECTRONIC DEVICE (20) |
|---|
| SECOND PROCESSOR (410) |
| SECOND COMMUNICATION CIRCUIT (420) |
| CAMERA (430) |
| TOUCH SCREEN (440) |
| SECOND MEMORY (450) |

# FIG.5

START

ACQUIRE PLURALITY OF IMAGES OF USER, CAPTURED BY RGB CAMERA(501)

IDENTIFY SPECIFIC AREAS FROM PLURALITY OF IMAGES (503)

GENERATE PLURALITY OF FIRST DATA ASSOCIATED WITH FIRST COLOR MODEL ON SPECIFIC AREAS OF PLURALITY OF IMAGES (505)

GENERATE PLURALITY OF SECOND DATA ASSOCIATED WITH SECOND COLOR MODEL BASED ON PLURALITY OF FIRST DATA (507)

GENERATE FIRST TIME-SERIES DATA ASSOCIATED WITH GREEN CHANNEL BASED ON PLURALITY OF FIRST DATA (509)

GENERATE SECOND TIME-SERIES DATA ASSOCIATED WITH COLOR DIFFERENCE CHANNEL BASED ON PLURALITY OF SECOND DATA (511)

GENERATE AT LEAST ONE INTEGRATED TIME-SERIES DATA ASSOCIATED WITH COMBINING FIRST TIME-SERIES DATA AND SECOND TIME-SERIES DATA (513)

ACQUIRE BIOMETRIC INFORMATION OF USER BASED ON INTEGRATED TIME-SERIES DATA (515)

END

# FIG.6

PROGRAM (300)

PLURALITY OF IMAGES

FACIAL AREA EXTRACTION
MODULE (610)

RGB DATA GENERATION
MODULE (620)

FIRST PRE-PROCESSING
MODULE (630)

TIME-SERIES DATA GENERATION MODULE (640)

| FIRST TIME-SERIES DATA GENERATION MODULE (640a) | SECOND TIME-SERIES DATA GENERATION MODULE (640b) | THIRD TIME-SERIES DATA GENERATION MODULE (640c) |

rPPG GENERATION
MODULE (650)

BIOMETRIC INFORMATION
ACQUISITION MODULE (660)

# FIG.7

| # | Frame#1 | Frame#2 | Frame#3 | Frame#4 | |
|---|---------|---------|---------|---------|---|
| B | 140 | 160 | 160 | 150 | ... |
| C | 120 | 140 | 130 | 150 | |
| D | 160 | 140 | 150 | 120 | |

TIME WINDOW (W1)

TIME WINDOW (W2)

⬇ AVERAGE CENTERING

| B | -13.33 | 5 | - | - | |
|---|--------|---|---|---|---|
| G | -10 | 5 | - | - | ... |
| R | 10 | -3.3 | - | - | |

# FIG.8

| B | -13.33 | 5 | - | - |
|---|--------|------|---|---|
| G | -10 | 5 | - | - |
| R | 10 | -3.3 | - | - |

. . .

| FIRST TIME-SERIES DATA GENERATION MODULE (640a) | SECOND TIME-SERIES DATA GENERATION MODULE (640b) | THIRD TIME-SERIES DATA GENERATION MODULE (640c) |
|---|---|---|
| GENERATE ((G-R)+(G-B)) VALUE TIME-SERIES DATA | YCrCb CONVERSION | CIE Lab CONVERSION |
| | GENERATE (Cr+Cb) VALUE TIME-SERIES DATA | GENERATE a VALUE TIME-SERIES DATA |

| FIRST TIME SERIES DATA (810) | SECOND TIME SERIES DATA (820) | THID TIME SERIES DATA (830) |
|---|---|---|

rPPG GENERATION MODULE (650)

EP 4 714 337 A1

*FIG.9*

START

ACQUIRE PLURALITY OF IMAGES OF USER,
CAPTURED BY IR CAMERA(901)

IDENTIFY PLURALITY OF AREAS OF FACE BASED
ON FACIAL FEATURE INFORMATION
EXTRACTED FROM PLURALITY OF IMAGES (903)

ACQUIRE PLURALITY OF TIME-SERIES DATA
ON IR CHANNEL FROM PLURALITY OF AREAS (905)

ACQUIRE INTEGRATED TIME-SERIES DATA BASED
ON AT LEAST ONE OF AVERAGE VALUE OR VARIANCE
VALUE OF PLURALITY OF TIME-SERIES DATA (907)

ACQUIRE BIOMETRIC INFORMATION OF USER
BASED ON INTEGRATED TIME-SERIES DATA (909)

END

29

# FIG.10

PROGRAM (300)

PLURALITY OF IMAGES

FEATURE POINT EXTRACTION
MODULE (1010)

LANDMARK AREA IDENTIFICATION
MODULE (1020)

LANDMARK AREA TIME-SERIES DATA
EXTRACTION MODULE (1030)

| FIRST AREA EXTRACTION MODULE (1030a) | SECOND AREA EXTRACTION MODULE (1030b) |
|---|---|
| THIRD AREA EXTRACTION MODULE (1030c) | FOURTH AREA EXTRACTION MODULE (1030d) |

PRE-PROCESSING MODULE (1040)

BIOMETRIC INFORMATION
ACQUISITION MODULE (1050)

## FIG.11

| # | Frame#1 | Frame#2 | Frame#3 | Frame#4 |
|----|-----------|-----------|-----------|-----------|
| L1 | 1-1 VALUE | 1-2 VALUE | 1-3 VALUE | 1-4 VALUE |
| L2 | 2-1 VALUE | 2-2 VALUE | 2-3 VALUE | 2-4 VALUE |
| L3 | 3-1 VALUE | 3-2 VALUE | 3-3 VALUE | 3-4 VALUE |
| L4 | 4-1 VALUE | 4-2 VALUE | 4-3 VALUE | 4-4 VALUE |

LANDMARK COMBINATION
MODULE (1100)

EP 4 714 337 A1

# FIG.12

START

ACQUIRE PHOTOGRAPHING
ENVIRONMENT CONDITION (1201)

IS MERGING
CONDITION SATISFIED?
(1203)

ACQUIRE FIRST
TIME-SERIES DATA BASED
ON RGB CAMERA (1205)

ACQUIRE SECOND
TIME-SERIES DATA BASED
ON IR CAMERA (1207)

ACQUIRE BIOMETRIC INFORMATION
BASED ON FIRST TIME-SERIES DAT
AND SECOND TIME-SERIES DATA (1209)

ACQUIRE TIME-SERIES
DATA BASED ON
IR CAMERA (1211)

ACQUIRE BIOMETRIC
INFORMATION BASED ON
TIME-SERIES DATA (1213)

END

FIG.13A

FIG.13B

FIG.13C

# FIG.14

START

ACQUIRE PLURALITY OF IMAGES OF USER,
CAPTURED BY RGB CAMERA(1401)

IDENTIFY SPECIFIC AREAS FROM
PLURALITY OF IMAGES (1403)

GENERATE PLURALITY OF FIRST DATA ASSOCIATED
WITH FIRST COLOR MODEL ON SPECIFIC
AREAS OF PLURALITY OF IMAGES (1405)

GENERATE PLURALITY OF SECOND DATA
ASSOCIATED WITH SECOND COLOR MODEL BASED
ON PLURALITY OF FIRST DATA (1407)

GENERATE FIRST TIME-SERIES DATA ASSOCIATED WITH
GREEN CHANNEL BASED ON PLURALITY OF FIRST DATA (1409)

GENERATE AT LEAST ONE SECOND TIME-SERIES
DATA ASSOCIATED WITH COLOR DIFFERENCE CHANNEL
BASED ON PLURALITY OF SECOND DATA (1411)

ACQUIRE BLOOD PRESSURE, BASED ON FIRST
TIME-SERIES DATA AND SECOND TIME-SERIES DATA
BEING INPUTTED TO BLOOD PRESSURE
MEASUREMENT AI MODEL (1413)

END

# FIG. 15

```
                    ┌─────────┐
                    │  START  │
                    └─────────┘
                         │
                         ▼
    ┌──────────────────────────────────────────────┐
    │     ACQUIRE PLURALITY OF IMAGES OF USER,      │
    │        CAPTURED BY RGB CAMERA(1501)           │
    └──────────────────────────────────────────────┘
                         │
                         ▼
    ┌──────────────────────────────────────────────┐
    │  GENERATE FIRST TIME-SERIES SIGNAL ASSOCIATED │
    │    WITH RGB COLOR MODEL ON SPECIFIC AREA,     │
    │      BASED ON PLURALITY OF IMAGES (1503)      │
    └──────────────────────────────────────────────┘
                         │
                         ▼
    ┌──────────────────────────────────────────────┐
    │ GENERATE SECOND TIME-SERIES SIGNAL ASSOCIATED │
    │ WITH CIE La*b* COLOR MODEL ON SPECIFIC AREA,  │
    │      BASED ON PLURALITY OF IMAGES (1505)      │
    └──────────────────────────────────────────────┘
                         │
                         ▼
    ┌──────────────────────────────────────────────┐
    │      CORRECT FIRST TIME-SERIES SIGNAL BASED   │
    │       ON SECOND TIME-SERIES SIGNAL (1507)     │
    └──────────────────────────────────────────────┘
                         │
                         ▼
    ┌──────────────────────────────────────────────┐
    │ GENERATE THIRD TIME-SERIES SIGNAL ASSOCIATED  │
    │      WITH YCrCgCb COLOR MODEL, BASED ON       │
    │   CORRECTED FIRST TIME-SERIES SIGNAL (1509)   │
    └──────────────────────────────────────────────┘
                         │
                         ▼
    ┌──────────────────────────────────────────────┐
    │      ACQUIRE STRESS INDEX ON USER BASED       │
    │          ON THIRD TIME-SERIES SIGNAL          │
    │      AND ADDITIONAL INFORMATION (1511)        │
    └──────────────────────────────────────────────┘
                         │
                         ▼
                    ┌─────────┐
                    │   END   │
                    └─────────┘
```

# FIG.16

PLURALITY OF IMAGE FRAMES

RGB DATA GENERATION
MODULE (1610)

CORRECTION MODULE (1620)

RGB INTERPOLATION

CORRECT RGB BASED ON
CIE La*b* CONVERSION
AND L CHANNEL

YCrCgCb TIME-SERIES
SIGNAL GENERATION
MODULE (1630)

PRE-PROCESSING
MODULE (1640)

HRV ANALYSIS
AI MODEL (1650)

rPPG EXTRACTION
MODULE (1670)

STRESS ANALYSIS
ALGORITHM (1660)

BIOMETRIC INFORMATION
ACQUISITION MODULE (1680)

EP 4 714 337 A1

# FIG.17

SERVER (10)

USER DEVICE (20)

TRANSPORTATION MEANS (V)

COMMUNICATION CIRCUIT (1710) | PROCESSOR (1720) | CAMERA (1730)

CONFIGURATION DEVICE (1740)

STEERING DEVICE (1740a) | ENGINE (1740b) | SEAT (1740c)

# FIG.18

START

IDENTIFY VEHICLE STARTING EVENT (1801)

CAPTURE PLURALITY OF IMAGES
OF USER BY USING CAMERA(1803)

ACQUIRE BIOMETRIC INFORMATION BASED
ON PLURALITY OF CAPTURED IMAGES (1805) → A

ACQUIRE INFORMATION ON EXTERNAL
ENVIRONMENT OF VEHICLE (1807)

CONTROL DEVICES IN VEHICLE BASED ON
BIOMETRIC INFORMATION AND INFORMATION → B
ON EXTERNAL ENVIRONMENT (1809)

END

*FIG.19A*

*FIG.19B*

# FIG.20

```
        ┌─────────┐
        │    A    │
        └─────────┘
             │
             ▼
┌─────────────────────────────────────┐
│ CAPTURE PLURALITY OF IMAGES ON PLURALITY │
│ OF PASSENGERS BY USING CAMERA (2001) │
└─────────────────────────────────────┘
             │
             ▼
┌─────────────────────────────────────┐
│   ACQUIRE BIOMETRIC INFORMATION OF   │
│   PLURALITY OF PASSENGERS BASED ON   │
│  PLURALITY OF CAPTURED IMAGES (2003) │
└─────────────────────────────────────┘
             │
             ▼
        ┌─────────┐
        │  1807   │
        └─────────┘
```

# FIG.21

Blood pressure 110 (Normal)

Blood pressure 130 (High)

## FIG.22

```
                    ( B )
                      |
                      v
  ┌─────────────────────────────────────────┐
  │  IDENTIFY THAT DRIVING MODE OF VEHICLE   │
  │   IS AUTONOMOUS DRIVING MODE (2201)      │
  └─────────────────────────────────────────┘
                      |
                      v
  ┌─────────────────────────────────────────┐
  │  CONTROL SHAPE OF SEAT OF VEHICLE BASED  │
  │     ON BIOMETRIC INFORMATION (2203)      │
  └─────────────────────────────────────────┘
                      |
                      v
  ┌─────────────────────────────────────────────┐
  │   CONTROL OTHER DEVICES IN VEHICLE BASED      │
  │ ON BIOMETRIC INFORMATION AND INFORMATION      │
  │   ON EXTERNAL ENVIRONMENT END (2205)          │
  └─────────────────────────────────────────────┘
                      |
                      v
                  ( END )
```

EP 4 714 337 A1

# FIG.23

Angle=A degree

Angle=B degree

Blood pressure value

E

Time

# FIG.24

```
                          ( START )
                             │
                             ▼
┌───────────────────────────────────────────────────────┐
│   IDENTIFY LIFE EVENT OF SPECIFIC TYPE (2401)          │
└───────────────────────────────────────────────────────┘
                             │
                             ▼
┌───────────────────────────────────────────────────────┐
│        CAPTURE PLURALITY OF IMAGES OF USER             │
│      BY USING CAMERA WHILE LIFE EVENT OF               │
│        SPECIFIC TYPE IS CONTINUED (2403)               │
└───────────────────────────────────────────────────────┘
                             │
                             ▼
┌───────────────────────────────────────────────────────┐
│  STORE BIOMETRIC INFORMATION IN FORM OF BEING          │
│  ASSOCIATED WITH ATTRIBUTE INFORMATION ON LIFE         │
│ EVENT, BASED ON PLURALITY OF CAPTURED IMAGES (2405)    │
└───────────────────────────────────────────────────────┘
                             │
                             ▼
┌───────────────────────────────────────────────────────┐
│ PROVIDE SERVICE BASED ON STORED INFORMATION (2407)     │
└───────────────────────────────────────────────────────┘
                             │
                             ▼
                          ( END )
```

# FIG.25A

Monday

84m

80

Sunday

114m

80

STORE BIOMETRIC
INFORMATION OF EACH
DRIVING ROUTE

DATABASE
(2500)

# FIG.25B

DATABASE
(2500)

PROVIDE DRIVING
RECOMMENDATION
INFORMATION

STRESS INDEX LV1

84m

80

Finish

STRESS INDEX LV3

# FIG.26

SERVER (10)

USER DEVICE (20)

KIOSK (2600)

COMMUNICATION CIRCUIT (2610) | PROCESSOR (2620) | CAMERA (2630)

DISPLAY (2640)

CONSTRUCTION SITE-RELATED INFORMATION (I)

EP 4 714 337 A1

# FIG.27

```
            ( START )
                │
                ▼
┌────────────────────────────────────────┐
│  ACQUIRE IDENTIFICATION INFORMATION     │
│      ON SPECIFIC USER (2701)            │
└────────────────────────────────────────┘
                │
                ▼
┌────────────────────────────────────────┐
│  IDENTIFY PLURALITY OF LOCATIONS WHERE  │
│   SPECIFIC USER IS POSITIONED (2703)    │
└────────────────────────────────────────┘
                │
                ▼
┌─────────────────────────────────────────────────┐
│  STORE BIOMETRIC INFORMATION OF SPECIFIC USER    │
│  ACCORDING TO PLURALITY OF LOCATIONS, BASED ON   │
│  PLURALITY OF IMAGES OF SPECIFIC USER CAPTURED BY│
│  USING KIOSKS PLACED AT PLURALITY OF LOCATIONS   │
│                    (2705)                        │
└─────────────────────────────────────────────────┘
                │
                ▼
┌────────────────────────────────────────┐
│  PROVIDE SERVICE BASED ON STORED        │
│  BIOMETRIC INFORMATION AT PLURALITY OF  │
│  LOCATIONS (2707)                       │
└────────────────────────────────────────┘
                │
                ▼
            ( END )
```

# FIG.28

2600c

THIRD CONSTRUCTION SITE

SERVER (10)

2600b

SECOND CONSTRUCTION SITE

2600a

FIRST CONSTRUCTION SITE

## FIG.29A

## FIG.29B

# EP 4 714 337 A1

Europäisches Patentamt
European Patent Office
Office européen des brevets

## EUROPEAN SEARCH REPORT

**Application Number**

EP 25 20 3266

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/153752 A1 (PARK KI BUM [KR]) 27 May 2021 (2021-05-27) | 1-3,5, 8-11,13 | INV. A61B5/00 |
| Y | * figure 4 * * paragraphs [0282] - [0284], [0290] - [0302], [0308] - [0310], [0511] - [0516], [0597] - [0598] * ----- | 6,14 | A61B5/024 A61B5/103 G06T7/00 G06V10/56 G06V40/10 |
| X | PANIGRAHI ARPITA ET AL: "Non-Contact HR Extraction from Different Color Spaces Using RGB Camera", 2022 NATIONAL CONFERENCE ON COMMUNICATIONS (NCC), IEEE, 24 May 2022 (2022-05-24), pages 332-337, XP034143390, DOI: 10.1109/NCC55593.2022.9806722 [retrieved on 2022-07-04] | 1-4, 7-12,15 | |
| Y | * figures 1,2 * * page 333, column 2, line 30 - page 335, column 1, line 31 * ----- | 6,14 | |

| | |
|---|---|
| | TECHNICAL FIELDS SEARCHED (IPC) |
| | A61B G06V G06T |

The present search report has been drawn up for all claims

| Place of search | Date of completion of the search | Examiner |
|---|---|---|
| The Hague | 5 November 2025 | Oancea, A |

EPO FORM 1503 03.82 (P04C01)

## ANNEX TO THE EUROPEAN SEARCH REPORT
## ON EUROPEAN PATENT APPLICATION NO.

EP 25 20 3266

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2025

| Patent document cited in search report | Publication date | Patent family member(s) | Publication date |
|---|---|---|---|
| US 2021153752 A1 | 27-05-2021 | US 2021153745 A1<br>US 2021153752 A1 | 27-05-2021<br>27-05-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240126409